(19) **Europäisches Patentamt / European Patent Office / Office européen des brevets**

(11) **EP 1 967 196 A1**

(12) **EUROPEAN PATENT APPLICATION**
published in accordance with Art. 158(3) EPC

(43) Date of publication:
**10.09.2008 Bulletin 2008/37**

(21) Application number: **06811295.2**

(22) Date of filing: **05.10.2006**

(51) Int Cl.:
*A61K 35/74* (2006.01)　　*A01N 63/00* (2006.01)
*A23K 1/16* (2006.01)　　*A23L 1/30* (2006.01)
*A61P 1/10* (2006.01)　　*A61P 31/04* (2006.01)
*A61P 43/00* (2006.01)　　*C12N 1/20* (2006.01)
*C12R 1/07* (2006.01)

(86) International application number:
**PCT/JP2006/319957**

(87) International publication number:
**WO 2007/058027 (24.05.2007 Gazette 2007/21)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.11.2005 JP 2005334553**
**16.02.2006 JP 2006039439**
**29.03.2006 JP 2006090766**
**05.04.2006 JP 2006104349**

(71) Applicant: **Idemitsu Kosan Co., Ltd.**
**Chiyoda-ku**
**Tokyo 100-8321 (JP)**

(72) Inventor: **MOCHIZUKI, Masami**
**Chiba 299-0293 (JP)**

(74) Representative: **Vossius & Partner**
**Siebertstrasse 4**
**81675 München (DE)**

(54) **HARMFUL BACTERIUM CONTROL AGENT CONTAINING BACILLUS THURINGIENSIS**

(57)　Through inhibition of the multiplication of pathogenic bacteria and inactivation of bacterial self-induced factors, the balance of intestinal flora is improved to thereby promote regulation of animal intestinal functions and enable prevention/treatment of infection disease caused by such pathogenic bacteria. Accordingly, a pharmaceutical containing *Bacillus thuringiensis* is administered to animals, and animals are allowed to ingest food or feed containing *Bacillus thuringiensis*.

EP 1 967 196 A1

**Description**

Technical Field

The present invention relates to a harmful bacterium control agent containing *Bacillus thuringiensis,* and also relates to a pharmaceutical, a food, and a feed each containing the harmful bacterium control agent.

Background Art

Each of microbial floras in a stomach and an intestine plays an extremely important many role in maintaining functions of a stomach and an intestinal tract and physiological health of an entire body. A probiotic therapy includes positive use of a microorganism which is beneficial for health. Living probiotics reach an intestine of a host, intensify a microbiologic flora in the intestine, and help a digestive activity of the intestine. Actions and properties of the probiotics such as "bacterial antagonistic action", "bacterial interference", "barrier effects", "settlement inhibition", and "competitive exclusion" improve a balance of a microbiologic flora in an intestinal tract and inhibit multiplication of a harmful line of microorganisms. Those probiotics cannot constantly form a colony in a digestive tract of a host. Thus, the probiotics needs to be periodically taken in for the sustentation of an action of the probiotics. In recent years, a lactic acid bacterium and a bifidobacterium have been widely used as probiotics, and those bacteria have been extensively utilized in yogurt and other dairy products. In addition, use of probiotic bacteria has also been expanding in industries such as livestock industries and pet industries, because the probiotic bacteria help a digestive activity of an animal, intensify intestinal bacteria, and inhibit multiplication of harmful intestinal bacteria, thereby resulting in promotion of growth of an animal. For use of bacteria as probiotics, bacteria are required to have resistance to gastric fluid, bile acid, and the like, have an ability to reach an intestine alive, have an ability to multiply not only in an upper section of a small intestine having an aerobic condition but also in a lower section of the small intestine and a large intestine both having an anaerobic condition, bring a useful action to a host, have an ability to preserve the bacteria themselves in a certain number or more in each form of a pharmaceutical and a food, be safe for an administration, etc.

In the background art described above, studies have been made on bacteria other than lactic acid bacteria and bifidobacteria, which can be used as probiotics. For example, it is reported that *Bacillus coagulans, Bacillus subtilis,* and *Bacillus clausii*, which have bile-acid resistance, are administered to mammals (Patent Document 1). As probiotics for human, *B. coagulans, B. subtilis, B. clausii, B. cereus, B. licheniformis, B*. pumilus, *B. polymyxa, B. vietnami,* and *B. polyfermenticus* are used (Non-patent Document 1). Further, bacteria such as those belonging to *Bacillus* (Patent Document 2, Patent Document 3, and Non-patent Document 2) and *Lactobacillus* (Patent Document 4), both having bile-acid resistance, are known.

On the other hand, there also exists problems of an intestinal infectious disease and an infectious disease infected from damaged skin. Pathogens causing those infectious diseases are always trying to find a niche place to settle in an animal body. Even in the case where pathogens invade in the animal body, the pathogens are usually removed by a biologic defense mechanism of a host, or multiplication of the pathogens is usually inhibited by other bacteria. On the contrary, there also exist some kinds of bacteria such as *Helicobacter pylori* and *Pseudomonas aeruginosa,* which multiply to a certain number of bacteria and settle in an intestine of an animal, thereby causing a chronic disease to a host. Those bacteria are not detected by a biologic defense mechanism of the host during the initial multiplication processes such as adhesion to skin, adhesion to mucosa, and invasion into an intestine. After sufficient bacterial density is attained, the bacteria produce various pathogenic factors at once to destroy tissues of the host. A mechanism of the actions described above is a cell-to-cell signaling mechanism via a self-induced factor produced by each of the bacteria themselves, namely a bacterial self-induced mechanism (bacterial quorum-sensing mechanism). The mechanism was found in a discovery of a phenomenon in which a fluorescent substance is produced depending on multiplication of bacteria in a culture of bacteria belonging to *Vibrio fischeri* (Non-patent Document 3). Afterward, the mechanism was found in many pathogens including *Pseudomonas aeruginosa.* The finding of the mechanism clarified the fact that an expression of a pathogenic factor is controlled by the mechanism. In addition, it is reported that many clinically important bacteria other than those belonging to *Pseudomonas* such as those belonging to *Burkholderia, Enterobacter, Vibrio, Serratia,* and *Salmonella* have the self-induced mechanism (Non-patent Document 4).

Signal transmission of the bacterial self-induced mechanism is carried out by a bacterial self-induced factor. As a bacterial self-induced factor of gram-negative bacteria, acyl homoserine lacton is extensively conserved. The bacterial self-induced factor is a molecule which can freely pass bacterial outer membrane. A concentration of the self-inducer factors is low in the case where bacterial density in an environment is low, so no biological activity is exhibited. On the other hand, with the increase of bacterial density in an environment by multiplication of bacteria, the concentration of the self-inducer factors is increased both inside and outside bacteria. When the concentration reaches a certain threshold value, the bacterial self-induced factors enhance an expression of a target gene for various pathogenic factors and the like.

As a method for prevention or treatment of infectious diseases caused by those pathogens, the following method is reported (Patent Document 5). A bacterial self-induced factor inactivation protein is orally administered to an animal. Alternatively, a nucleic acid sequence encoding the bacterial self-induced factor inactivation protein is introduced into animal cells to express the nucleic acid sequence. The bacterial self-induced factor inactivation protein inactivates the bacterial self-induced factor of the pathogens, whereby the production of the pathogenic factor is inhibited. However, the method of orally administrating the bacterial self-induced factor inactivation protein has a problem that the protein is digested and decomposed in the process of passing through a stomach, thereby not reaching an intestinal tract. Further, it is difficult to attain introduction of nucleic acid sequence in the human and in grown-up non-human animals.

In addition, there exists problems in *Clostridium perfringens, Staphylococcus aureus, Bacillus cereus,* and *Streptococcus suis*. For example, *Clostridium perfringens* and *Staphylococcus aureus* are pathogens causing food poisoning to a human or causing diarrhea to poultry and livestock. *Bacillus cereus* is a pathogen causing food poisoning to a human. *Streptococcus suis* is a pathogen causing a nervous symptom to a human or causing diarrhea and a nervous symptom to poultry and livestock. Those bacteria are pathogens belonging to a gram-positive bacterium. Countermeasures for the infectious diseases caused by those pathogens are demanded.

As bacteria having antagonistic property to gram-positive bacteria, bifidobacterium effective for treatment of *Clostridium difficile* related diarrhea (Patent Document 6), and B. subtilis having an antibacterial action to harmful bacteria each belonging to *Staphylococcus* or *Clostridium* (Patent Document 7) is reported. However, those bacteria do not work sufficiently to prevent or treat the infectious diseases described above. Under the circumstances of the above-mentioned background art, novel bacterium species have been searched as probiotics, which are excellent in a multiplication ability in a digestive tract and effective for prevention and treatment of a wide range of infectious diseases caused by pathogens belonging to a gram-positive bacterium.

It is reported that *Bacillus thuringiensis* inhibits quorum-sensing dependent pathogenicity of *Erwinia carotovora* which is a pathogen for a plant (Non-patent Document 5). Specifically, it is reported that *Bacillus thuringiensis* produces AHL-lactonase which is an enzyme for decomposing a bacterial quorum-sensing signal factor (acyl homoserine lacton (AHL)) to inhibit accumulation of AHL in an environment. Such *Bacillus thuringiensis* is widely taken in an agricultural production as a safe microorganism pesticide, but administration thereof to animals including a human has not been investigated. In addition, it is reported that a crystalline protein produced by *Bacillus thuringiensis* has a pesticidal activity against a nematode, a trematode, and a protozoa which parasitize in a plant host or an animal host (Patent Document 8 and Patent Document 9). However, those documents only disclose that *Bacillus thuringiensis* has pesticidal property to the nematode and the like separated from an animal's intestine, and do not confirm the effect and safety of actual oral administration of *Bacillus thuringiensis* to animals including a human.

That is, no investigation has been made at all until now on that infectious diseases caused by pathogens are prevented or treated by using a pharmaceutical containing *Bacillus thuringiensis,* and that the human ingestion of a food containing *Bacillus thuringiensis* or animals' ingestion of a feed containing *Bacillus thuringiensis* inhibits multiplication of harmful bacteria living in an intestinal tract and improve a balance of an intestinal flora to prevent or treat the infectious diseases.

[Patent Document 1] JP 2005-507670 A

[Patent Document 2] JP 05-268944 A

[Patent Document 3] WO 96/024659

[Patent Document 4] JP 2004-523241 A

[Patent Document 5] JP 2003-504028 A

[Patent Document 6] JP 2005-508617 A

[Patent Document 7] JP 11-285378 A

[Patent Document 8] JP 2002-34582 A

[Patent Document 9] US 5,468,483

[Non-patent Document 1] Senesi, S., Bacillus Spores as Probiotic Products for Human Use, Bacterial spore formers, 2004, pp. 131-141, Rica, E., Henriques, A.O., and Cutting, S.M.(eds), Horizon Bioscience, Wymondham Norfolk NR18 OJA U.K.

[Non-patent Document 2] Hyronimus, B. et al., International Journal of Food Microbiology, 2000, 61, pp. 193-197

[Non-patent Document 3] Kaplan,H. et al., Journal of Bacteriology, 1985, 163, pp. 1210-1214

[Non-patent Document 4] Tateda,K., The Lung Perspectives, 2005, 13, pp. 261-265

[Non-patent Document 5] Yi-Hu Dong et al., Applied and Environmental Microbiology, 2004, 70, pp. 954-960

Disclosure of the Invention

It is an object of the present invention to provide means for improving a balance of an intestinal flora, promoting an intestinal function, and preventing and treating an infectious disease by inhibiting multiplication of a harmful bacterium, the means being able to be used for an animal including a human safely and simply. Specifically, an object of the present invention is to provide a pharmaceutical for prevention and treatment of an infectious disease caused by a pathogen by administration thereof to a human, poultry, livestock, and the like. Another object of the present invention is to provide a food and a feed for inhibiting multiplication of a harmful bacterium to improve a balance of an intestinal flora and to thereby prevent and treat an infectious disease by regular ingestion of the food or feed.

To achieve the above-mentioned objects, the inventors of the present invention sought a bacterial species capable of controlling a harmful bacterium and studied effectiveness of the bacterial species and safety thereof to an animal. As a result, the inventors found that *Bacillus thuringiensis* had an ability to inhibit multiplication of a harmful bacterium and useful property as probiotics.

Thus, the inventors found the following facts: a spore of a bacterium belonging to *Bacillus thuringiensis* is orally administered; the bacterial spore passes through a stomach alive, and then germinates and multiplies in an intestine without being killed by a bile acid; and the multiplied bacteria inhibit multiplication of a pathogen to improve a balance of an intestinal flora and to prevent and treat an infectious disease in an intestine.

That is, the present invention is as follows.

(1) a harmful bacterium control agent, comprising *Bacillus thuringiensis*;

(2) a harmful bacterium control agent according to the item (1), in which the *Bacillus thuringiensis* has a bile acid resistance;

(3) a harmful bacterium control agent according to the item (1) or (2), in which the *Bacillus thuringiensis* further has an ability of bacterial self-induced factor inactivation;

(4) a harmful bacterium control agent according to any one of the items (1) to (3), in which the *Bacillus thuringiensis* is a *Bacillus thuringiensis* subsp. *thuringiensis* BGSC 4A3 strain, a *Bacillus thuringiensis* subsp. *kurstaki* BGSC 4D1 strain, a *Bacillus thuringiensis* subsp. *aizawai* BGSC 4J4 strain, a *Bacillus thuringiensis* subsp. *israelensis* BGSC 4Q7 strain, or mutants thereof;

(5) a harmful bacterium control agent according to any one of the items (1) to (4), in which the harmful bacterium is a pathogen causing an infectious disease;

(6) a harmful bacterium control agent according to any one of the items (1) to (5), in which the pathogen is a pathogen belonging to a gram-negative bacterium;

(7) a harmful bacterium control agent according to the item (6), in which the pathogen belonging to a gram-negative bacterium includes at least any one of bacteria each belonging to genus *Salmonella, Campylobacter, Vibro, Yersinia, Aeromonas, Pseudomonas, Escherichia coli,* and *Shigella;*

(8) a harmful bacterium control agent according to any one of the items (1) to (5), in which the pathogen is a pathogen belonging to a gram-positive bacterium;

(9) a pathogen control agent according to the item (8), in which the pathogen belonging to a gram-positive bacterium includes at least any one of bacteria each belonging to genus *Clostridium, Bacillus, Staphylococcus, Streptococcus,* and *Listeria;*

(10) a pharmaceutical comprising the harmful bacterium control agent according to any one of the items (1) to (9);

(11) a pharmaceutical according to the item (10), in which the pharmaceutical is for preventing or treating an infectious disease caused by a pathogen;

(12) a pharmaceutical according to the item (10) or (11), in which the pharmaceutical is for human;

(13) a pharmaceutical according to the item (10) or (11), in which the pharmaceutical is for a non-human animal;

(14) a food comprising the harmful bacterium control agent according to any one of the items (1) to (9);

(15) a feed comprising the harmful bacterium control agent according to any one of the items (1) to (9);

(16) a feed according to the item (15), in which the feed is for preventing or treating an intestinal infectious disease of an animal; and

(17) a method of breeding an animal comprising the step of feeding the feed according to the item (15) or (16) to an animal.

In addition, the present invention provides a use of *Bacillus thuringiensis* in a production of the harmful bacterium control agent and a harmful bacterium-controlling method comprising administrating the *Bacillus thuringiensis* to an animal requiring the control of harmful bacteria. A preferable embodiment of the use and method is the same as in the case of the harmful bacterium control agent.

Best Mode for carrying out the Invention

The harmful bacterium control agent of the present invention is characterized by including *Bacillus thuringiensis.* In the present invention, the harmful bacterium is referred to as fungi and bacteria adversely effecting on animals by proliferating inside a digestive organ such as an intestine of the animal. For example, bacteria which cause dyspepsia, diarrhea, loose stool, constipation, or the like by proliferating inside the intestine, and pathogens which cause an intestinal infectious disease by producing pathogenic factors inside the intestine are exemplified. The inhibition of the harmful bacterial proliferation corresponds to inhibiting the colonization and the proliferation of harmful bacteria in animal bodies. The proliferation is inhibited, for example, by an antagonism, a bacterial interference, or a barrier effect with a *Bacillus thuringiensis* bacterium, or by the inhibition of the colonization to an intestinal tract cell or competitive elimination of harmful bacteria with a *Bacillus thuringiensis* bacterium.

The *Bacillus thuringiensis* used in the harmful bacterium control agent of the present invention is a bacterium classified into *"Bacillus thuringiensis"* in Bergey's Manual of Determinative Bacteriology, 9th edition (1994).

The subspecies of the *Bacillus thuringiensis* used in the harmful bacterium control agent of the present invention are not particularly limited. For example, *Bacillus thuringiensis* subsp. *thuringiensis, Bacillus thuringiensis* subsp. *kurstaki, Bacillus thuringiensis* subsp. *aizawai,* and *Bacillus thuringiensis* subsp. *israelensis* are preferably used. Of those, *Bacillus thuringiensis* subsp. *thuringiensis* BGSC 4A3 strain, *Bacillus thuringiensis* subsp. *kurstaki* BGSC 4D1 strain, *Bacillus thuringiensis* subsp. *aizawai* BGSC 4J4 strain, *Bacillus thuringiensis* subsp. *israelensis* BGSC 4Q7 strain, and the like are preferably used. BGSC 4A3, BGSC 4D1, BGSC 4J4, and BGSC 4Q7 are strains registered in Bacillus Genetic Stock Center (BGSC) (The Department of Biochemist).

In addition, the mutants of BGSC 4A3, BGSC 4D1, BGSC 4J4, and BGSC 4Q7 may be used in the harmful bacterium control agent of the present invention. The mutants to be used may be one in which the above each strain underwent a natural mutation or one obtained by mutating each strain with a chemical mutation agent, UV rays, or the like. Of those mutants, preferably used is a mutant having at least one of properties selected from a bile acid resistance, a bacterial self-induced factor inactivation, a disinfection activity, an anaerobically facultative property, and an acid resistance, which are the same as in each strain. Those microbiological characteristics will be described later. Further, a mutant having the same microbiological characteristics as each strain in addition to the above microbiological characteristics is also preferably used. In addition, respective mutants of the above strains exhibiting the same control ability for harmful bacteria as each strain is also preferably used.

It is preferable that the *Bacillus thuringiensis* used in the harmful bacterium control agent of the present invention have a bile acid resistance.

The phrase "have an bile acid resistance" corresponds to form a spore having an ability of germinating and proliferating in a medium containing a bile acid at high concentration.

The bile acid is referred to as a steroid containing four-ring structure, which is found widely in the bile of mammalians, birds, reptiles, amphibians, and fishes. Examples of the bile acid include cholic acid, chenodeoxycholic acid, deoxycholic acid, lithocholic acid, and ursodeoxycholic acid. In animal bodies, the bile acid is generally present as conjugated one binding to glycine or taurine with an amide bound in bile to form a sodium salt thereof. In the specification of the present invention, in a case of indicating simply "bile acid," , the bile acid includes the above-mentioned bile acid, salts thereof, and conjugated one thereof.

As the medium containing a bile acid at high concentration, exemplified is a medium which contains a bile powder (Oxgall, manufactured by Difco Laboratories) obtained by condensing and exsiccating a fresh bile by 10 folds and has a bile powder concentration of 0.3% by mass or more, preferably 1% by mass or more, and more preferably 3% by mass or more. In addition, the phrase "have an ability of germinating and proliferating" corresponds to a case where bacteria germinate and restart proliferating and division to form colonies when the spores are inoculated in the medium containing a bile acid at high concentration as described above and conditions except for the bile acid concentration is set to one suitable for the culture of *Bacillus thuringiensis.*

A *Bacillus thuringiensis* having a bile acid resistance can be obtained as follow. An isolated specimen containing *Bacillus thuringiensis* is cultured under a condition suitable for a spore formation to form a spore. The spore thus obtained is inoculated to the medium added with bile acid at high concentration, and cultured, whereby formed colonies are isolated. Of those colonies, a colony having mycological characteristics of *Bacillus thuringiensis* is selected.

The *Bacillus thuringiensis* used in the harmful bacterium control agent of the present invention preferably has a bacterial self-induced factor inactivation ability. "Bacterial self-induced factor" is a signal molecule playing a role of an intercellular transmission in a bacterial self-induced mechanism. The signal molecule promotes an expression of a gene producing a specific substance when the bacteria are proliferated. As the bacterial self-induced factor, exemplified are N-butanoyl-L-homoserine lactone (BHL), N-(3-hydroxybutanoyl)-L-homaserine lactone (HBHL), N-(3-oxobutanoyl)-L-homoserine lactone (OBHL), N-hexanoyl-L-homoserine lactone (HHL), N-(3-oxohexanoyl)-L-homoserine lactone (OHHL), N-octanoyl-L-homoserine lactone (OHL), N-(3-oxooctanoyl)-L-homoserine lactone(OOHL), N-(3-oxodecanoyl)-L-homoserine lactone (ODHL), and N-(3-oxododecanoyl)-L-homoserine lactone (OdDHL). The phrase "have a bacterial self-induced factor inactivation ability" corresponds to, for example, have an ability of producing a enzyme degrading a bacterial self-induced factor. The phrase "producing an enzyme" corresponds to producing an enzyme to such an extent that an enzyme activity can be detected in the medium when bacterial cells are cultured.

In addition, the phrase "have a bacterial self-induced factor inactivation ability" corresponds to inhibit the production of a specific substance such as a pathogenic factor induced by a bacterial self-induced factor.

The bacterial self-induced factor inactivation of *Bacillus thuringiensis* is confirmed, for example, by the following method involving: culturing *Bacillus thuringiensis* as a sample in a culture solution containing a bacterial self-induced factor; adding the culture solution to a medium to which a bacterium having a bacterial self-induced mechanism is inoculated; culturing the bacterium for a predetermined period; and confirming with an appropriate marker whether the bacterium having a bacterial self-induced mechanism produces or not a specific substance by an influence of the bacterial self-induced factor. In a case where a specific substance is detected, it is understood that a bacterial self-induced factor is not inactivated, while in a case where no specific substance is detected, it is understood that the bacterial self-induced factor is inactivated and a bacterial self-induced mechanism is not induced. As the bacterium having a bacterial self-induced mechanism, for example, a bacterial species that produces a pigment when a bacterial self-induced factor amount reaches a predetermined concentration may be used. There is, for example, a method involving: culturing *Bacillus thuringiensis* as a sample in a culture solution containing OHHL for a predetermined period; adding the culture solution to a medium where a bacterial self-induced factor biosynthetic gene-deleted strain of *Chromobacterium violaceum* synthesizing a violet pigment (violacein) by the bacterial self-induced mechanism grows; and testing whether a violacein induction is attained or not (McClean,K. et al, Microbiology, 1997,143, pp. 3073-3711).

Further, the *Bacillus thuringiensis* used in the harmful bacterium control agent of the present invention is preferably a facultative anaerobe which can proliferate under an anaerobic condition. The anaerobic condition means the condition with an oxygen concentration equal to or less than that in the gas contained in animal intestines. In a laboratory, the anaerobic condition means the condition with an oxidation-reduction potential of -10 mV or less, preferably -50 mV or less when measured at 20°C. The oxidation-reduction potential can be measured with a generally-used oxidation-reduction electrometer available in the market. The digestive tract of the human is in a microaerobic or anaerobic condition, so the bacterium can proliferate satisfactorily even in the intestinal tract condition.

The *Bacillus thuringiensis* used in the harmful bacterium control agent of the present invention also preferably has an acid resistance. By using such a bacterium, even inside the stomach, the bacterium reaches the intestine without dying. The phrase "have an acid resistance" corresponds to a case where the bacterium is administered to an animal and reaches the intestine without dying under a stomach inside condition (in a state of taking foods, normally, with a pH of 3.5 to 6) and a number of bacterial cells are maintained to such an extent that the bacterium can proliferate if the bacterium reaches the intestine. The spore of *Bacillus thuringiensis* has generally an acid resistance, so there is no problem in a case of using the spore.

The fact that *Bacillus thuringiensis* bacterium reaches the intestine without dying inside the stomach is confirmed, for example, by measuring a bacterial cell concentration in feces.

The method of culturing *Bacillus thuringiensis* used in the harmful bacterium control agent of the present invention is not particularly limited, and the *Bacillus thuringiensis* can be cultured by a conventional method under an appropriate condition according to bacterial characteristics. For example, the bacterium may be cultured at a culture temperature of 15 to 45°C, preferably at 20 to 42°C, and more preferably 25 to 38°C. In addition, as the culture method, a liquid culture method or a solid culture method by a static culture, reciprocating shaking culture, a rotary shaking culture, or a jar fermenter culture may be used.

The medium component to be used in the culture is not limited. As a carbon source, saccharides such as glucose, galactose, lactose, arabinose, mannose, sucrose, starch, starch hydrolysate, and molasses, organic acids such as citric acid, and alcohols such as glycerin may be used. As a nitrogen source, ammonium salts and nitrates such as ammonia, ammonium sulfate, ammonium chloride, and ammonium nitrate may be used. In addition, inorganic salts such as sodium chloride, potassium chloride, potassium phosphate, magnesium sulfate, calcium chloride, calcium nitrate, manganese chloride, and ferrous sulfate, and peptone, a soybean flour, a defatted soybean cake, meat extract, and yeast extract may be used.

The *Bacillus thuringiensis* used in the harmful bacterium control agent of the present invention is preferably in a form of spore in terms of storage stability and acid resistance. The *Bacillus thuringiensis* in the form of spore has high resistance to heat and drying, so it can be dried satisfactorily when pharmaceuticals, foods, or feeds are produced, with a result that the storage stability is enhanced. In order to make the bacterium form the spore, the culture conditions such as medium composition, pH of medium, culture temperature, culture humidity, and oxygen concentration during the culture may be adjusted to a condition for spore formation in a period of culture. As a culture method for the spore formation, for example, there is a method described in Schaeffer, P., J. Millet, J.P. Aubert, Proceedings of the National Academy of Science, US, 1965, 54, pp. 704 to 711.

The culture or bacterial cells thus obtained by the above method is preferably formed into dry powders in terms of storage property. It is preferred to dry them so that a water content of the harmful bacterium control agent becomes 20% by mass or less.

The dry method is not particularly limited, and there are given a natural drying, a forced-air drying, a spray drying, a freeze drying, and the like. Of those, a spray drying and a forced-air drying are preferably used. A protective agent such as skim milk, sodium glutamate, and saccharides may be used in a time of drying. As saccharides, glucose and trehalose may be used. Further, after drying, it is preferred to place the dried product in a gas-barrier aluminum bag with a deoxidant and a dehydrating agent added, seal the bag, and preserve it at a room temperature to a lower temperature. With the method, a living bacterium can be preserved for a long time.

The harmful bacterium control agent of the present invention is preferably used for particularly controlling a pathogen causing an infectious disease among the harmful bacteria. Examples of the pathogen include pathogens which belong to gram-negative bacteria causing an intestinal infectious disease, such as bacteria belonging to genus *Vibrio, Salmonella, Campylobacter, Yersinia,* or *Aeromonas, Escherichia coli,* and *Shigella.* Examples of the bacteria belonging to genus *Vibrio* include *V. parahaemolyticus* and *V. cholerae.* Examples of the bacteria belonging to genus *Salmonella* include *S. typhimurium, S. enteritidis,* S. *pullorum, S. gallinarum, S. typhisuis, S. chorelaesuis, S. derby, and S. dublin.* Examples of the bacteria belonging to genus *Campylobacter* include *C. jejuni, C.* coli, and *C. fetus.* Examples of the bacteria belonging to genus *Yersinia* include *Y. enterocolitica* and Y. *pseudotuberculosis.* Examples of the bacteria belonging to genus *Aeramonas* include *A. hydrophila, A. caviae, A. veronii, A. jandaei,* and *A. schubertii.* Examples of *Escherichia coli* include *enteroinvasive E. coli* (EIEC), *enterotoxigenic E. coli* (ETEC), *enteropathogenic E. coli* (EPEC), *Shiga toxin producing E. coli* (STEC), and *Enteroaggregative E. coli* (EAEC). Examples of *Shigella* include *S. dysenteriae, S. flexneri, S. boydii, and S. sonnei.*

In addition, as a pathogen proliferating in the skin, cuticle, mucosa, or the like and causing an infectious disease, there are given bacteria belonging to genus *Pseudmonas.* An example of the bacteria includes *P. aeruginosa.*

In addition, the harmful bacterium control agent of the present invention is preferably used for pathogens belonging to a gram-positive bacterium. Examples of the pathogens include bacteria belonging to genus *Clostridium, Bacillus, Staphylococcus,* genus *Streptococcus,* and genus *Listeria.* Examples of bacteria belonging to genus *Clostridium* include *C. perfringens, C. difficile, and C. botulinum.* An Example of bacteria belonging to genus Bacillus includes *B. cereus.* An example of bacteria belonging to genus *Staphylococcus* includes *S. aureus.* Examples of bacteria belonging to genus *Streptococcus* include *S. suis* and *S. pyogenes.*

The harmful bacterium control agent of the present invention can be used as a pharmaceutical for controlling a harmful bacterium by adding, as required, appropriate components such as a carrier and an bulking agent generally used in pharmaceuticals and forming the harmful bacterium control agent into a formulation. The pharmaceutical of the present invention is particularly preferably used for preventing and treating an infectious disease caused by pathogens. A dosage form of the pharmaceutical can be selected as required according to a site where the pharmaceutical is administered, a disease to be targeted for prevention and treatment, and the like. Hereinafter, specific use and dosage form of the pharmaceutical will be described.

The pharmaceutical of the present invention may be used as an intestinal regulator for improving the balance of intestinal flora or used for preventing and treating an intestinal infectious disease caused by pathogens. In this case, examples of the dosage form of the pharmaceutical include solid formulations such as a tablet, a granule, a powder, and a capsule, a water soluble powder, and syrup each for an oral administration, and an injection, suppository, and percutaneous absorption agent each for a parenteral administration. In addition, the pharmaceutical may be formed into a water soluble powder for administration by enema for a colonic cleaning. In this case, the water soluble powder can be produced by mixing *Bacillus thuringiensis* and a physiological saline.

In addition, the pharmaceutical of the present invention may be used for inhibiting the proliferation of the harmful bacterium in the skin or mucosa and preventing and treating infectious diseases of the skin or mucosa.

To be specific, the pharmaceutical of the present invention may be used for inhibiting a proliferation of cavity bacteria, thereby preventing dental caries, periodontal diseases such as gingivitis, and halitosis. In this case, examples of the dosage form of the pharmaceutical preferably include a tablet capable of being masticated, a toothpaste, a mouthwash, and a cavity drop.

In addition, the pharmaceutical of the present invention may be also used for controlling pathogens in the skin or the cuticle and particularly preferably used for promoting cure of a skin injury. In this case, examples of the dosage form of the pharmaceutical include an ointment, a cream, a spray, a lotion, a gel, and a patch.

In addition, the pharmaceutical of the present invention may be used for improving an intravaginal flora or preventing and treating an intravaginal infection of pathogens. By improving the intravaginal flora, it is expected to prevent a yeast infection in the vagina and a bacterial vaginal disease. In this case, examples of the dosage form of the pharmaceutical include a cream, a lotion, and a gel.

The pharmaceutical of the present invention may be also used for an inflammation of eyes, nasal cavity, and ears, and the like. The dosage form in this case can be also selected as required.

In addition, the pharmaceutical of the present invention may further include another component having a function of inhibiting a proliferation of a harmful bacterium in addition to *Bacillus thuringiensis.* The component is not particularly limited as long as the component does not lead to dying of *Bacillus thuringiensis* and the safety to animals has been confirmed.

The bacterial cell concentration of *Bacillus thuringiensis* used in the pharmaceutical of the present invention may be set to a concentration suitable for administrating an effective amount of *Bacillus thuringiensis* according to the intended use and dosage form of the pharmaceutical. In general, the concentration may be set to a range of $1 \times 10^4$ to $1 \times 10^{12}$ CFU/g, preferably $1 \times 10^5$ to $1 \times 10^{11}$ CFU/g, and more preferably $1 \times 10^6$ to $1 \times 10^{10}$ CFU/g.

The pharmaceutical of the present invention may be administered to the human and non-human animals. The administration may be performed with an appropriate method in order to administer an effective amount of *Bacillus thuringiensis* according to the dosage form and intended use of the pharmaceutical, and the age, sex, body weight, and physical condition of an administration target, and state of disease at an applying site. For example, in a case where the pharmaceutical is orally administered for the purpose of promoting a regulation of an intestinal function or preventing and treating an intestinal infectious disease, in general, the *Bacillus thuringiensis* is preferably administered in a range of $1 \times 10^5$ to $1 \times 10^{10}$ CFU/kg of body weight/day. In addition, in a case of using the pharmaceutical in external use for the purpose of controlling pathogens in the skin, the cuticle, or the mucosa, the pharmaceutical amount can be appropriately adjusted according to an application range, and it is generally preferred to administer *Bacillus thuringiensis* in a range of $1 \times 10^5$ to $1 \times 10^{10}$ CFU/10 to 1,000 mg/day.

The harmful bacterium control agent of the present invention may be formed into a food having an improving effect of an intestinal flora balance or controlling effect of a regulation of an intestinal function, or a food having a preventing and treating effect of an intestinal infectious disease by adding and mixing the control agent in a beverage and food or blending the control agent with an arbitrary component generally used in a beverage and food, thereby processing them. A form of each of the arbitrary component and the food is not particularly limited as long as bacterial cells of *Bacillus thuringiensis* do not die, and they are generally used in foods. For example, the food can be processed into a supplement such as a drinkable preparation, a powder, a tablet, or a capsule. In addition, the pharmaceutical may be used in breads, noodles, confectioneries such as a biscuit, a chocolate, and a candy, tea bags, a hot cereal such as oatmeal, soups, hot beverages, sweeteners, flavoring agents, spices, an instant coffee, a dried product or freeze-dried product such as a dried cream, meat-processing products such as ham and sausage, and fish-processing products such as a fish cake and a boiled flat fish cake. The food of the present invention is particularly preferably formed into a form of a supplement in terms of storage stability.

A bacterial cell concentration of the *Bacillus thuringiensis* in the food of the present invention may be selected according to a form of the food as long as the concentration is in a range suitable for administration of the effective amount of *Bacillus thuringiensis.* For example, the concentration is preferably, in general, set to one suitable for administrating *Bacillus thuringiensis* in the range of $1 \times 10^5$ to $1 \times 10^{10}$ CFU/kg of body weight/day. For such a concentration, the bacterial cell concentration of *Bacillus thuringiensis* is set to, for example, $1 \times 10^4$ to $1 \times 10^{12}$ CFU/g, preferably $1 \times 10^5$ to $1 \times 10^{11}$ CFU/g, and more preferably $1 \times 10^6$ to $1 \times 10^{10}$ CFU/g.

In addition, the food of the present invention may be produced by a conventional method of food processing except for mixing the harmful bacterium control agent of the present invention. When the harmful bacterium of the present invention is, for example, in a state of powder or solid, the control agent may be also used by forming into a liquid or a gel for easy mixing with the food component. In this case, water, vegetable oils such as a soybean oil, a rape oil, and a corn oil, liquid animal oils, water-soluble polymer compounds such as polyvinyl alcohol, polyvinyl pyrrolidone, and polyacrylic acid can be used as a liquid carrier. In addition, in order to maintain the uniformity of the bacterial cell concentration in the food, it is also preferred to blend a water-soluble polysaccharide such as arginic acid, sodium arginate, xanthan gum, sodium casein, an arabic rubber, a guar gum, and tamarind seed polysaccharides. In order to prevent a multiplication of saprophyte, an organic acid such as propionic acid, ascorbic acid, or citric acid is blended to produce an acidic liquid viable-cell agent.

The harmful bacterium control agent of the present invention may be used for a feed additive to be added and mixed in a feed. The feed additive can be used by being added and mixed in a feed. In addition, the harmful bacterium control agent of the present invention may be formed into a feed for improving an intestinal flora balance, promoting a regulation of intestinal function, and promoting growth (increase in body weight) or a feed for preventing and treating an intestinal infectious disease by being processed with an arbitrary component generally used in feeds. In this case, the arbitrary component and a form of feed is not particularly limited as long as no bacterial cells of *Bacillus thuringiensis* die, and the component or form generally used in feeds for animals, such as feeds for livestock, pet foods, and supplements for animals, can be used. The content of the harmful bacterium control agent in the feed of the present invention is not limited as long as the content is in a range suitable for ingestion of effective amount of *Bacillus thuringiensis,* and the content may be adjusted, as required, depending on the kind, body weight, age, sex of the animal, use purpose, health condition of the animal to be administered, and the feed component. For example, the content is preferably, in general, set to one suitable for ingesting *Bacillus thuringiensis* in the range of $1 \times 10^5$ to $1 \times 10^{10}$ CFU/kg of body weight/day. For such a concentration, the bacterial cell concentration of *Bacillus thuringiensis* is set to a range of, for example, $1 \times 10^4$ to $1 \times 10^{12}$ CFU/g, preferably $1 \times 10^5$ to $1 \times 10^{11}$ CFU/g, and more preferably $1 \times 10^6$ to $1 \times 10^{10}$ CFU/g.

The feed of the present invention can be produced by mixing the harmful bacterium control agent of the present invention as it is. However, when the harmful bacterium control agent in a state of powder or solid is added and mixed into the feed, the agent formed into a liquid or gel may also be used as in a food processing.

The kind of animal to be ingested with the feed of the present invention is not particularly limited, and mammalians, birds, reptiles, amphibians, and fishes are exemplified. Of those, the feed of the present invention can be preferably used for poultry selected from fowls, ducks, quails, and turkeys, and livestock selected from pigs, cows, sheep, and rabbits. A feed amount to be ingested in the animal may be adjusted, as required, depending on the kind, body weight, age, and sex of the animal, the use purpose, the health condition of the animal, the feed component, and the like. The method of preventing and treating an intestinal infectious disease of animals includes feeding to the animal the feed of the present invention obtained by blending the harmful bacterium control agent of the present invention into the feed component. The administration method and a breeding method of animals may be conducted with a method generally used in breeding animals.

Examples

(1) Test of bile acid resistance and anaerobic property

To 1,000 ml of deionized water was added 35 g of a normal agar medium "Nissui" (manufactured by Nissui Pharmaceutical Co., Ltd.), and the whole was sterilized in an autoclave. After sterilization, the mixture was cooled to 45°C, and bile powder (Oxgall, manufactured by Difco Laboratories) was sterilized by filtration using a cellulose-mixed ester type membrane filter (pore size 0.45 $\mu$m, manufactured by Advantec Toyo Kaisha, Ltd.) and added to the mixture to prepare bile powder-supplemented normal agar plate media with bile powder concentrations of 0.3% by mass, 1% by mass, and 3% by mass.

BGSC 4A3, BGSC 4D1, BGSC 4J4, and BGSC 4Q7 belonging to *Bacillus thuringiensis* were separately inoculated into a bile powder-nonsupplemented normal agar plate medium (1,000 ml of deionized water, 35 g of "Nissui") and cultured at 37°C overnight. After culture, bacterial cells were separated from colonies formed on the plate medium and inoculated into the bile powder-supplemented normal agar plate media prepared above using pre-sterilized disposable loops. The cells were cultured at 37°C for 2 days to confirm whether colonies were formed or not (Examples 1 to 4).

In order to confirm multiplication abilities of BGSC 4A3, BGSC 4D1, BGSC 4J4, and BGSC 4Q7 under anaerobic conditions, the respective bacterial strains were inoculated into the bile powder-nonsupplemented normal agar plate medium prepared above and cultured under anaerobic conditions at 37°C for 2 days using Anaeropack Kenki (manufactured by Mitsubishi Gas. Chemical Co., Ltd.) to confirm whether colonies were formed or not.

On the other hand, *Bacillus coagulans* ATCC8038, *Bacillus coagulans* NBRC12583, *Bacillus coagulans* DSM2312, *Bacillus subtilis* NBRC3009, *Bacillus subtilis* NBRC3025, *Bacillus subtilis* NBRC3108, *Bacillus subtilis* NBRC3336, *Bacillus clausii* DSM2512, *Bacillus clausii* DSM2515, *Bacillus clausii* DSM2525, and *Bacillus clausii* DSM8716 were separately inoculated into the bile powder-supplemented media in the same way as above and cultured at 37°C for 2 days. Meanwhile, multiplication tests under anaerobic conditions were performed in the same way as above (Comparative Examples 1 to 11).

ATCC8038 is a strain deposited in the American Type Culture Collection (ATCC); NBRC12583, NBRC3009, NBRC3025, NBRC3108, and NBRC3336 are strains deposited in the Biological Resource Center of the National Institute of Technology and Evaluation (NBRC); and DSM2312, DSM2512, DSM2515, DSM2525, DSM8716 are strains deposited in the Deutsche Sammlung von Mikroorganismen und Zellkulturen GmbH (DSMZ).

The results are shown in Table 1.

Table 1

| | Strain | Anaerobic culture | Bile powder content in normal agar medium (%) | | | |
| --- | --- | --- | --- | --- | --- | --- |
| | | | 0 | 0.3 | 1 | 3 |
| Example 1 | Bacillus thuringiensis subsp. thuringiensis BGSC 4A3 | + | + | + | + | + |
| Example 2 | Bacillus thuringiensis subsp. kurstaki BGSC 4D1 | + | + | + | + | + |
| Example 3 | Bacillus thuringiensis subsp. aizawai BGSC 4J4 | + | + | + | + | + |
| Examples 4 | Bacillus thuringiensis subsp. israelensis BGSC 4Q7 | + | + | + | + | + |

(continued)

| | Strain | Anaerobic culture | Bile powder content in normal agar medium (%) | | | |
|---|---|---|---|---|---|---|
| | | | 0 | 0.3 | 1 | 3 |
| Comparative Example 1 | Bacillus coagulans ATCC8038 | + | + | ± | - | - |
| Comparative Example 2 | Bacillus coagulans NBRC12583 | + | + | ± | - | - |
| Comparative Example 3 | Bacillus coagulans DSM2312 | + | + | ± | - | - |
| Comparative Example 4 | Bacillus subtilis NBRC3009 | - | + | + | + | + |
| Comparative Example 5 | Bacillus subtilis NBRC3025 | - | + | + | + | + |
| Comparative Example 6 | Bacillus subtilis NBRC3108 | - | + | + | + | + |
| Comparative Example 7 | Bacillus subtilis NBRC3336 | - | + | + | + | + |
| Comparative Example 8 | Bacillus clausii DSM2512 | - | + | + | + | + |
| Comparative Example 9 | Bacillus clausii DSM2515 | - | + | + | + | + |
| Comparative Example 10 | Bacillus clausii DSM2525 | - | + | + | + | + |
| Comparative Example 11 | Bacillus clausii DSM8716 | - | + | + | + | + |
| +: Formation of colonies was observed. ±: Formation of only a few colonies was observed. -: Formation of colonies was not observed. | | | | | | |

It was confirmed that *Bacillus thuringiensis* strains of Examples 1 to 4 can form colonies on all the normal agar plate media with bile powder concentrations of 0.3% by mass, 1% by mass, and 3% by mass, and can multiply even under anaerobic conditions.

On the other hand, although *Bacillus coagulans* strains of Comparative Examples 1 to 3 were confirmed to multiply under anaerobic conditions, they formed only a few colonies on the normal agar medium with a bile powder concentration of 0.3% by mass and formed no colony on the media with bile powder concentrations of 1% by mass and 3% by mass, and they were found to be inferior in bile acid resistance to *Bacillus thuringiensis* strains of Examples 1 to 4. In addition, *Bacillus subtilis* strains of Comparative Examples 4 to 7 were found to have bile acid resistance but have no ability to multiply under anaerobic conditions. *Bacillus clausii* DSM2512, DSM2515, DSM2525, DSM8716 of Comparative Examples 8 to 11 were found to have bile acid resistance but have no ability to multiply under anaerobic conditions.

(2) Test of bacterial self-induced factor inactivation ability

*Chromobacterium violaceum* CV026 with a disrupted bacterial self-induced factor-producing gene was used to perform a test for confirming induction of a bacterial self-induced mechanism (McClean, K. et al., Microbiology, 143, 3703-3711 (1997)). The CV026 strain is a strain with a disrupted biosynthetic gene of N-hexanoyl-L-homoserine lactone (HHL), which is a bacterial self-induced factor, and in the case where an acyl homoserine lactone (AHL) such as HHL is not added form the outside, the strain cannot express a bacterial self-induced mechanism and cannot synthesize a violet pigment (violacein). Also in the case where N-(3-oxohexanoyl)-L-homoserine lactone (OHHL) is added as an AHL instead of HHL, the strain can express a bacterial self-induced mechanism. A specific method of the test is as follows.

*Chromobacterium violaceum* CV026 was inoculated into Luria-Bertani (LB) medium and cultured with shaking at 28°C overnight. Subsequently, 50 μl of the CV026 culture solution obtained above and 50 μl of an aqueous solution of 2% kanamycin sterilized by filtration were added to 5 ml of a semifluid LB agar medium (0.3%) sterilized in an autoclave and cooled to 45°C, and 5 ml of the semifluid LB agar medium was layered on LB agar medium, which was prepared in advance in a petri dish with a diameter of 9 cm. The agar was solidified, and holes with a diameter of 6 mm were formed in the LB agar medium using a cork borer.

CV026 is a strain deposited in the National Collection of Type Cultures (NCTC) as NCTC13278.

To 10 ml of LB medium was added 0.5 ml of 500 μM OHHL (manufactured by Sigma), and the whole was allowed to stand at 28°C for 4 hours and used as a positive control. Meanwhile, LB medium supplemented with no additive was treated in the same way as above and used as a negative control. The controls were separately added in an amount of 50 μl to the holes of the LB medium containing CV026 prepared above, culture was performed at 28°C for 24 hours. As a result, in the medium where the positive control containing OHHL was added, induction of violacein was observed around the holes of the medium, while in the medium where the negative control containing no OHHL was added, induction of violacein was not observed. The results reveal that CV026 can express a bacterial self-induced mechanism and can induce violacein when cultured in the presence of OHHL but cannot express a bacterial self-induced mechanism and cannot induce violacein when cultured in the absence of OHHL.

Subsequently, in order to examine the OHHL inactivation ability of *Bacillus bacteria, Bacillus* bacteria was allowed to react with OHHL in accordance with the following procedure.

*Bacillus thuringiensis* BGSC 4A3, BGSC 4D1, BGSC 4J4, BGSC 4Q7 (Examples 1 to 4), and *Bacillus coagulans, Bacillus subtilis,* and *Bacillus clausii* shown in Table 2 were separately inoculated into LB medium and cultured with shaking at 37°C overnight (Comparative Examples 1 to 11). Thereafter, 0.5 ml of OHHL was added to 10 ml of each culture solution with an absorbance (660 nm) adjusted to 1.0, followed by a reaction at 28°C for 4 hours. Each of the reaction solutions was added in an amount of 50 μl to the holes of the LB medium containing CV026, and culture was performed at 28°C for 24 hours. After culture, induction of violacein around each hole was observed.
The results are shown in Table 2.

Table 2

|  | Strain | Induction of violacein |
|---|---|---|
| Positive control | - (Supplemented with OHHL) | + |
| Negative control | - (Supplemented with no OHHL) | - |
| Example 1 Example 1 | Bacillus thuringiensis subsp. thuringiensis BGSC 4A3 | - |
| Example 2 | Bacillus thuringiensis subsp. kurstaki BGSC 4D1 | - |
| Example 3 | Bacillus thuringiensis subsp. aizawai BGSC 4J4 | - |
| Example 4 | Bacillus thuringiensis subsp. israelensis BGSC 4Q7 | - |
| Comparative Example 1 | Bacillus coagulans ATCC8038 | + |
| Comparative Example 2 | Bacillus coagulans NBRC12583 | + |
| Comparative Example 3 | Bacillus coagulans DSM2312 | + |
| Comparative Example 4 | Bacillus subtilis NBRC3009 | + |
| Comparative Example 5 | Bacillus subtilis NBRC3025 | + |
| Comparative Example 6 | Bacillus subtilis NBRC3108 | + |
| Comparative Example 7 | Bacillus subtilis NBRC3336 | + |
| Comparative Example 8 | Bacillus clausii DSM2512 | + |
| Comparative Example 9 | Bacillus clausii DSM2515 | + |
| Comparative Example 10 | Bacillus clausii DSM2525 | + |
| Comparative Example 11 | Bacillus clausii DSM8716 | + |
| +: Induction of violacein was observed. -: Induction of violacein was not observed. | | |

In all the strains of *Bacillus thuringiensis* of Examples 1 to 4, the induction of violacein was not observed. On the other hand, in all the strains of *Bacillus coagulans* of Comparative Examples 1 to 3, *Bacillus subtilis* of Comparative Examples 4 to 7, and *Bacillus clausii* of Comparative Examples 8 to 11, the induction of violacein was observed. The results reveal that the *Bacillus thuringiensis* strains can produce an OHHL-degrading enzyme to inactivate OHHL and to inhibit expression of the bacterial self-induced mechanism of CV026, while the *Bacillus* bacteria of Comparative Examples 1 to 11 cannot inactivate OHHL.

(3) Production of harmful bacterium control agent

The medium for spore formation, having the following composition, was used to culture BGSC 4A3, BGSC 4D1, BGSC 4J4, and BGSC 4Q7 at 37°C for 72 hours in a liquid (Proceedings of the National Academy of Sciences, US, 1965, 54, pp. 704-711). The resultant culture solutions were centrifuged to collect bacterial cells. The collected cells were freeze-dried and pulverized, and lactose was added thereto so that the density of spores reached $5 \times 10^8$ CFU/g to produce harmful bacterium control agents, which were used in Production Examples 1 to 4, respectively. The spore density was measured by: diluting each of the harmful bacterium control agents produced above to an appropriate concentration with sterilized water; heating the agent at 70°C for 30 minutes to kill only vegetative cells; inoculating the cells into a normal agar medium; and counting the number of colonies formed thereon.

(Component of medium for spore formation)

| | |
|---|---|
| Nutrient broth (manufactured by Difco Laboratories) | 8.0 g |
| KCl | 1.0 g |
| $MgSO_4 \cdot 7H_2O$ | 0.25 g |
| $MnCl_2 \cdot 4H_2O$ | 0.002 g |
| Total, adjusted to pH 7.0 | 1,000 ml |

The components above were sterilized in an autoclave, and a $CaCl_2$ solution and a $FeSO_4$ solution, which had been pre-sterilized, were added so as to be in amounts of $5 \times 10^{-4}$ M and $1 \times 10^{-6}$ M, respectively.

On the other hand, compositions produced in the same way as above using *Bacillus coagulans* NBRC12583 and *Bacillus subtilis* NBRC3009 designate Comparative Production Examples 1 and 2, respectively.

(4) Chick growth test

Feeds obtained by mixing the harmful bacterium control agents of Production Examples 1 to 4 at a concentration of 0.2% by mass with respect to the total mass of a feed for chick (for SD broiler in early and later stages, manufactured by Nippon Formula Feed Mfg Co., Ltd., a feed supplemented with no antibacterial substance) were used in Examples 5 to 8. Chicks of the respective groups, each group consisting of 12 chicks hatched from hatching eggs derived from broiler chickens (species: Chunky), were allowed to ingest the feeds of Examples 5 to 8 for 56 days to perform a growth test. On the other hand, a feed obtained by mixing the compositions of Comparative Production Examples 1 and 2 at a concentration of 0.2% were used in Comparative Examples 12 and 13. A feed obtained by mixing lactose instead of a harmful bacterium control agent at a concentration of 0.2% was designated Control Example and used in a growth test in the same way as above.

56 days later, the body weights of the chicks of the respective groups were measured, and mean values for the respective groups were calculated. Meanwhile, the cell concentrations of the added bacteria in the stools during the breeding period were measured, and mean concentrations for the respective groups were calculated.

The results are shown in Table 3.

Table 3

| | Amount of agent added (based on dry feed) (%) | Chick mean body weight at the time of start (g) | Chick mean body weight 56 days later (g) | Cell concentration of added bacterium in stool (CFU/g) |
|---|---|---|---|---|
| Example 5 | 0.2 | 50 | 3,184 | $12.1 \times 10^6$ |
| Example 6 | 0.2 | 50 | 3,101 | $11.3 \times 10^6$ |
| Example 7 | 0.2 | 50 | 3,157 | $20.9 \times 10^6$ |
| Example 8 | 0.2 | 50 | 3,074 | $17.6 \times 10^6$ |
| Comparative Example 12 | 0.2 | 50 | 2,686 | $0.1 \times 10^6$ |
| Comparative Example 13 | 0.2 | 50 | 2,824 | $0.2 \times 10^6$ |
| Control Example | 0.2 | 50 | 2,769 | 0 |

The body weights of the chicks fed with the feeds of Examples 5 to 8 were more increased compared with those of the chicks fed with the feeds of Comparative Examples 12 and 13. In the stools of the chicks fed with the feeds of Examples 5 to 8, the added bacteria were detected at concentrations of 11.3 × 10⁶ CFU/g (gram of raw chick stool) or more, and the numbers of the bacterial cells were significantly larger than those in the stools of the chicks fed with the feeds of Comparative Examples 12 and 13. The results reveal that the harmful bacterium control agents containing spores of *Bacillus thuringiensis* of the present invention can promote the body weight gain of an animal because the bacterium is not killed in a digestive tract such as stomach or intestine and forms colonies in the intestine.

(5) Pig feeding test

Feeds obtained by mixing the harmful bacterium control agents of Production Examples 1 and 3 with a standard feed for pig (Premium Power, manufactured by Showa Sangyo Co., Ltd.) were used in Examples 9 and 10, respectively, and baby pigs of the respective groups, each group consisting of 30 pigs, were allowed to ingest the feeds. In the same way as above, a feed obtained by mixing the composition of Comparative Production Example 1 with the standard feed for pig was designated Comparative Example 14, and baby pigs were allowed to ingest the feed. A feed obtained by mixing lactose instead of a harmful bacterium control agent was designated Control Example, and baby pigs were allowed to ingest the feed.

21 days later, the body weights of the baby pigs of the respective groups were measured, and mean values for the respective groups were calculated. In addition, the occurrence of diarrhea or loose stool during the period was observed, and the days when one baby pig developed diarrhea or loose stool were counted and summed as one point per day. The results were evaluated as diarrhea or loose stool scores.

The results are shown in Table 4.

Table 4

|  | Amount of agent added (based on dry feed) (%) | Mean body weight of pigs at the time of start (kg) | Mean body weight of pigs 21 days later (kg) | Diarrhea or loose stool score |
|---|---|---|---|---|
| Example 9 | 0.5 | 18.0 | 32.9 | 4 |
| Example 10 | 0.5 | 17.9 | 32.4 | 7 |
| Comparative Example 14 | 0.5 | 18.0 | 31.8 | 39 |
| Control Example | 0.5 | 18.0 | 31.4 | 61 |

The body weights of the baby pigs fed with the feeds of Examples 9 and 10 were more increased compared with those of the baby pigs fed with the feed of Comparative Example 14. Meanwhile, in the cases of the baby pigs fed with the feeds of Examples 9 and 10, the diarrhea or loose stool scores were significantly lower than the score of the baby pigs fed with the feed of Comparative Example 14. The results suggest that the harmful bacterium control agents containing spores of *Bacillus thuringiensis* of the present invention can promote the body weight gain of baby pigs and have ability to prevent or ameliorate diarrhea or loose stool.

(6) Calf breeding test

One-week-old male calves (Holstein) of the respective groups, each group consisting of 6 calves, were bred. Feeds obtained by mixing the harmful bacterium control agents at a concentration of 2% by mass of Production Examples 1 and 3 with a substitute milk shown below were used in Examples 11 and 12, respectively, while a feed obtained by mixing the composition of Comparative Production Example 1 at a concentration of 2% by mass was designated Comparative Example 15. A feed obtained by mixing lactose in the same amount as above was designated Control Example. The calves were allowed to ingest the substitute milk and a feed for calf in suckling period (Ushikko, manufactured by Snow Brand Seed Co., Ltd.) to raise them until the calves became 35-day-old, and the occurrence of diarrhea or loose stool during the period was observed.

(Substitute milk composition)

|  | (% by mass) |
|---|---|
| Non-fat dry milk | 69 |
| Whey | 10 |
| Beef tallow | 12.2 |
| Lecitin | 0.8 |
| Vitamins, minerals, and other additives | 8 |

(Evaluation criterion of occurrence of diarrhea or loose stool)

Frequently: Total number of calves that developed diarrhea in the test period is 10 or more.

Scarcely: Total number of calves that developed diarrhea in the test period is less than 10 and 5 or more.

Very scarcely: Total number of calves that developed diarrhea in the test period is less than 5.

The results are shown in Table 5

Table 5

| | Amount of agent added (based on dry feed) (%) | Mean body weight of calves at the time of start (kg) | Mean body weight of calves 28 days later (kg) | Occurrence of diarrhea or loose stool |
|---|---|---|---|---|
| Example 11 | 2.0 | 49.8 | 70.2 | Very scarcely |
| Example 12 | 2.0 | 50.0 | 70.8 | Very scarcely |
| Comparative Example 15 | 2.0 | 49.9 | 69.6 | Scarcely |
| Control Example | 2.0 | 50.1 | 69.0 | Frequently |

The body weights of the calves fed with the substitute milks of Examples 11 and 12 were more increased compared with those of the calves fed with the substitute milk of Comparative Example 15. Meanwhile, in the cases of the calves fed with the substitute milks of Examples 11 and 12, the diarrhea or loose stool scores were significantly lower than the score of the calves fed with the substitute milk of Comparative Example 15. The results suggest that the harmful bacterium control agents containing spores of *Bacillus thuringiensis* of the present invention can promote the body weight gain of calves and have ability to prevent or ameliorate diarrhea or loose stool.

The results of growth/breeding of chicks, baby pigs, and calves reveal that, if a feed containing a harmful bacterium control agent of the present invention is given, *Bacillus thuringiensis* is colonized in the intestine at a high frequency to improve digestion and absorption of the feeds and to promote the growth, and to prevent/ameliorate of an undesirable symptom such as diarrhea or loose stool.

(7) Test of effect of improving milk quality

Feeds obtained by mixing the harmful bacterium control agents of Production Examples 1 and 3 with a feed for dairy cow (4.5 kg of corn, 2.5 kg of barley, 1.1 to 1.8 kg of beet, 1.8 kg of cotton seed, 1.4 to 1.6 kg of defatted soybean, 0.6 kg to 1.2 kg of soybean, 10 to 12 kg of dry grass, and 160 to 220 L of water) were used in Examples 13 and 14, respectively, and separately given to dairy cows (Holstein) of the respective groups, each group consisting of 30 cows. A feed obtained by mixing the composition of Comparative Production Example 1 with a feed for dairy cow was designated Comparative 16 and given in the say way as above. In addition, a feed obtained by mixing lactose instead of a harmful bacterium control agent was designated Control Example and given in the same way as above.

For the dairy cows of the respective groups, milking was performed at intervals of 10 days 17 times, and the milk fat and milk protein ratios and the no-fat milk solid ratio of each milk were measured to calculate mean values.

The results are shown in Table 6.

Table 6

| | Amount of agent added (based on dry feed) (%) | Milk fat ratio (%) | Milk protein ratio (%) | No-fat milk solid ratio (%) |
|---|---|---|---|---|
| Example 13 | 0.5 | 4.10 | 3.39 | 9.01 |
| Example 14 | 0.5 | 3.99 | 3.42 | 8.99 |
| Comparative Example 16 | 0.5 | 3.92 | 3.26 | 8.84 |
| Control Example | 0.5 | 3.89 | 3.18 | 8.79 |

The milk fat content, milk protein content, and no-fat milk solid content in each milk produced by the dairy cows fed with the feeds of Examples 13 and 14 were found to be larger than those in each milk produced by the dairy cows fed with the feed of Comparative Example 16. The results reveal that the harmful bacterium control agents containing spores of *Bacillus thuringiensis* of the present invention has ability to improve the milk quality of a dairy cow.

(8) Challenge study of *Salmonella enteritidis* to chick

Feeds obtained by mixing the harmful bacterium control agents of Production Examples 1 to 4 at a concentration of 0.2% by mass with respect to the total mass of a feed for chick (for SD broiler in early stage, manufactured by Nippon Formula Feed Mfg Co., Ltd., a feed supplemented with no antibacterial substance) were used in Examples 15 to 18, respectively. Chicks of the respective groups, each group consisting of 12 chicks hatched from hatching eggs derived from broiler chickens (species: Chunky), were allowed to ingest the feeds of Examples 15 to 18 for 12 days. On the other hand, feeds obtained by mixing the compositions of Comparative Production Examples 1 and 2 at a concentration of 0.2% by mass were used in Comparative Examples 17 and 18. A feed obtained by mixing lactose instead of a harmful bacterium control agent at a concentration of 0.2% by mass was designated Control Example and fed to the chicks in the same way as above. When the chicks became 7-day-old, the chicks were orally challenged by *Salmonella enteritidis* (SE) at $1.2 \times 10^7$ CFU/chick. And when the chicks became 12-day-old, the cecal content and the stool were collected from each chick. The stool were collected by wiping the cloacae with swabs. The SE used was a strain separated from the cecal content of a chicken that had died at a farm of a poultry producer in Gunma prefecture.

The SE viable cells in the cecal contents were counted by the following method to calculate infection indices and defense indices.

1 g of each cecal content was diluted 10-fold with sterilized phosphate buffered saline, and the solution was mixed well to prepare a sample stock solution. Subsequently, the sample stock solution was serially diluted 10-fold with sterilized physiological saline to prepare serially-diluted solutions. The sample stock solution and serially-diluted solutions were separately smeared on SS agar plate medium "Nissui" (manufactured by Nissui Pharmaceutical Co., Ltd.) and a brilliant green agar plate medium (manufactured by Difco Laboratories) in an amount of 0.1 ml and cultured at 37°C for 24 hours, and the number of typical SE colonies multiplied on each plate medium was measured. Moreover, bacterium cells were collected from the colonies and inoculated into SIM agar medium for lysine decarboxylation test "Nissui" (manufactured by Nissui Pharmaceutical Co., Ltd.) and TSI agar medium "Nissui" (manufactured by Nissui Pharmaceutical Co., Ltd.), and they were cultured at 37°C for 24 hours to confirm their properties.

The number of SE viable cells in 1 g of the cecal content was calculated by multiplying the number of colonies confirmed to be SE by a dilution factor of the diluted solution. Based on the results, infection indices and defense indices were calculated as follows. The infection index is a value indicating the infection rate of a pathogen, while the defense index is a value indicating the ability of each feed to protect the infection of a pathogen, which is compared with the case of administration of a feed not containing a *Bacillus* bacterium.

Infection index: mean value of logarithms of the numbers of SE viable cells in cecal contents of the respective individuals (mean value of log CFU/g)

Defense index: infection index of control group/infection index of each test group

For each stool collected from the cloacae, qualitative culture was performed by the following method to observe the properties of SE. That is, whether SE colonies were detected or not in each individual was confirmed by: suspending the stool attached to a swab in 10 ml of sterilized phosphate buffered saline to prepare a sample stock solution; smearing the solution in an amount of 0.1 ml on SS agar medium and a brilliant green agar plate medium; and performing culture at 37°C for 24 hours.

Moreover, it was determined if homoserine lactone is present in the cecal contents of the respective groups collected above.

First, the following test was performed to examine a relationship between the concentration of homoserine lactone and induction of a bacterial self-induced mechanism.

*Chromobacterium violaceum* CV026 was inoculated into LB medium and cultured with shaking at 28°C overnight. Subsequently, 50 μl of the CV026 culture solution obtained above and 50 μl of an aqueous solution of 2% kanamycin sterilized by filtration were added to 5 ml of a semifluid LB agar medium (0.3%), which had been sterilized in an autoclave and cooled to 45°C, and 5 ml of the semifluid LB agar medium was layered on LB agar medium which was previously prepared in a petri dish with a diameter of 9 cm. The agar was solidified, and 50 μl of 500 μM, 50 μM, or 5 μM OHHL (manufactured by Sigma) was added dropwise to the surface of the LB medium layered with CV026 strain and dried for 30 minutes, followed by culture at 28 °C for 24 hours. As a result, in the medium where 500 μM or 50 μM OHHL was added dropwise, induction of violacein was observed around the region where OHHL was added dropwise, while in the medium where 5 μM OHHL was added dropwise, induction of violacein was not observed around the region where OHHL was added dropwise.

Thereafter, the stock solution of each cecal content in sterilized phosphate buffered saline prepared above was centrifuged at 4°C for 30 minutes at 20,000 × g, and a double volume of dichloromethane (special grade, manufactured by Wako Pure Chemical Industries, Ltd.) was added to the resultant supernatant, followed by shaking for 1 hour to perform extraction. The extraction was repeated twice. The organic layers were combined, and water was removed with anhydrous sodium sulfate (special grade, manufactured by Wako Pure Chemical Industries, Ltd.). Then, evaporation was performed to remove the solvent, and 50 μl of sterilized water was added thereto. The thus-prepared solution of the cecal content of each group was added dropwise in an amount of 50 μl on the surface of the LB medium layered with CV026 and dried for 30 minutes, and culture was performed at 28°C for 24 hours. After culture, induction of violacein was observed around the region where the above-described solution was added dropwise.

Meanwhile, the bacterial cell concentrations of added *Bacillus thuringiensis* in the stool during the breeding period were measured, and the mean concentrations were calculated for the respective groups.

The results are shown in Table 7.

Table 7

| | Number of viable cells in cecal content | | Number of individuals whose stools, collected from cloacae, contain SE Number of test chicks (12) | Induction of violacein in cecal content | Cell concentration of added *Bacillus* bacterium in stool ($\times 10^6$ CFU/g) |
|---|---|---|---|---|---|
| | Infection index | Defense index | | | |
| Example 15 | 1.2 | 7.1 | 2 | - | 16.3 |
| Example 16 | 2.1 | 4.0 | 3 | - | 9.9 |
| Example 17 | 0.8 | 10.6 | 1 | - | 18.5 |
| Example 18 | 1.9 | 4.5 | 2 | - | 11.0 |
| Comparative Example 17 | 4.4 | 1.9 | 8 | + | 0.2 |
| Comparative Example 18 | 5.1 | 1.7 | 9 | + | 0.3 |
| Control Example | 8.5 | - | 12 | + | 0.0 |
| +: Induction of violacein was observed. -: Induction of violacein was not observed. | | | | | |

In the case of the chicks administered with the feeds containing *Bacillus thuringiensis* of Examples 15 to 18, the bacterial cell concentrations of SE in the cecal contents were extremely low, and the infection indices of the *Salmonella* bacterium were extremely low. In addition, a visual inspection of the viscera of the chicks showed that there are few bleeding lesions in the cecum. As is clear from the defense indices, the infection indices were found to be about 1/4 to 1/10 compared with Control Example.

In the cases of the chicks fed with the feeds of Comparative Examples 17 and 18, multiplication of SE was slightly inhibited compared with Control Example. A visual inspection of the viscera of the chicks showed that there are bleeding lesions in the cecum at a high frequency. In the stools of the chicks fed with the harmful bacterium control agents of Example 15 to 18, the added bacterium was detected at $9.9 \times 10^6$ CFU/g (gram of raw chick stool) or more, and the numbers of viable cells were significantly larger compared with Comparative Examples.

The results reveal that the harmful bacterium control agents for animals containing spores of *Bacillus thuringiensis* of the present invention can inhibit multiplication of SE and can inhibit expression of bacterial self-induced factors because the strain is not killed in a digestive tract such as stomach or intestine and forms colonies in the intestine. That is, addition of spores of a bacterium *Bacillus thuringiensis* of the present invention to a feed is effective in prevention/treatment of intestinal infectious diseases caused by SE.

(9) Challenge study of *Escherichia coli* to pig

Feeds obtained by mixing the harmful bacterium control agents of Production Examples 1 and 3 at a concentration of 0.5% by mass with respect to the total mass of a feed for swine (Premium Series, manufactured by Showa Sangyo Co., Ltd.) were used in Examples 19 and 20, respectively, and pigs of the respective groups, each group consisting of 30 pigs (35-day-old), were allowed to ingest the feeds for 19 days. In the same way as above, a feed obtained by mixing the harmful bacterium control agent of Comparative Production Example 1 at a concentration of 0.5% by mass was designated Comparative Example 19 and ingested to the pigs. In addition, a feed obtained by mixing lactose instead of a harmful bacterium control agent at a concentration of 0.5% by mass was designated Control Example and ingested to the pigs. When the pigs became 40-day-old, the pigs were orally challenged by an edema disease bacterium (*Es-*

*cherichia coli*) at $1.8 \times 10^5$ CFU/pig. The edema disease bacterium used was a strain separated from the bowel content of a pig, which had developed edema disease and died in a hog farm in Kitakyusyu. The pigs were bred until the pigs became 54-day-old, and the death rates of pigs of the respective group were calculated.

In addition, the number of viable cells of *E. coli* in the small intestine content was measured by the following method. 1 g of the small intestine content was diluted 10-fold with sterilized phosphate buffered saline, and the whole was mixed well to prepare a sample stock solution. Subsequently, the sample stock solution was serially diluted 10-fold with sterilized physiological saline. The sample stock solution and serially-diluted solutions were separately smeared on DHL agar plate medium (granule) "Nissui" (manufactured by Nissui Pharmaceutical Co., Ltd.) in an amount of 0.1 ml, and culture was performed at 37°C for 24 hours, followed by measurement of the number of typical colonies of *E. coli* multiplied on each plate medium. The number of *E. coli* viable cells in 1 g of the small intestine content was calculated by multiplying the number of colonies confirmed to be *E. coli* by a dilution factor of the diluted solution, and the infection index was calculated by the following method.

Infection index: mean value of logarithms of the numbers of *E. coli* viable cells in small intestines of the respective individuals (mean value of log CFU/g)

Moreover, in order to examine the presence or absence of homoserine lactone in the small intestine content, induction of violacein was observed by the same method as that described in the item (8) using CV026 strain.
The results are shown in Table 8.

Table 8

|  | Death rate 14 days after infection of edema disease bacterium (%) | Number of viable cells in small intestine content (log CFU/g) (infection index) | Induction of violacein in small intestine content |
|---|---|---|---|
| Example 19 | 10 | 0.9 | - |
| Example 20 | 6 | 1.1 | - |
| Comparative Example 19 | 43 | 5.9 | + |
| Control Example | 60 | 8.2 | + |
| +: Induction of violacein was observed. -: Induction of violacein was not observed. | | | |

The death rate of Control Example and the death rate of the test group of Comparative Example 19 were found to be 60% and 43%, respectively, and an edema disease phase such as eyelid edema or torticollis were observed in dead pigs. In surviving pigs, loss of vitality or rough fur was observed. In the cases of the pigs fed with the harmful bacterium control agents containing *Bacillus thuringiensis* of Examples 19 and 20, the death rates were found to be 10% or less.

In the cases of Examples 19 and 20, multiplication of *E. coli* was inhibited in the small 1. intestine contents of the pigs. In addition, induction of bacterial self-induced factors was inhibited. The results reveal that administration of the harmful bacterium control agents containing spores of *Bacillus thuringiensis* of the present invention to pigs is effective in prevention/treatment of infection of edema disease.

(10) Challenge study of *Salmonella* to calf One-week-old male calves (Holstein) of the respective groups, each group consisting of 8 calves, were bred. Feeds obtained by mixing the harmful bacterium control agents of Production Examples 1 and 3 at a concentration of 2.0% by mass with a mixed feed for calf (Miracle Mate, manufactured by Scientific Feed Laboratory Co., Ltd.) were used in Examples 21 and 22, respectively, while a feed obtained by mixing the composition of Comparative Production Example 1 at a concentration of 2.0% was designated Comparative Example 20. In addition, a feed obtained by adding lactose instead of a harmful bacterium control agent in an equal amount was designated Control Example. These mixed feeds for calf were ingested to the calves until the calves became 4-week-old. When the calves became 2-week-old, the calves were orally challenged by a *Salmonella typhimurium* (ST) at $8.9 \times 10^5$ CFU/calf. The ST used was a strain separated from a fresh stool of a cow, which had developed diarrhea in the farm of a dairy farmer in Kitakyusyu. The calves were bred until the calves became 4-week-old, and the death rates of the calves of the respective groups were calculated.

In addition, the number of viable cells of ST in the small intestine content collected was measured by the following method. 1 g of the small intestine content was diluted 10-fold with sterilized phosphate buffered saline, and the whole was mixed well to prepare a sample stock solution. Subsequently, the sample stock solution was serially diluted 10-fold with sterilized physiological saline. The sample stock solution and serially-diluted solutions were separately smeared on SS agar plate medium "Nissui" (manufactured by Nissui Pharmaceutical Co., Ltd.) and a brilliant green agar plate medium (manufactured by Disco Laboratories) in an amount of 0.1 ml, and culture was performed at 37°C for 24 hours, followed by measurement of the number of typical colonies of ST multiplied on each plate medium. The bacterium was collected from the colonies and inoculated into SIM agar medium for lysine decarboxylation test "Nissui" (manufactured by Nissui Pharmaceutical Co., Ltd.) and TSI agar medium "Nissui" (manufactured by Nissui Pharmaceutical Co., Ltd.), and culture was performed at 374°C for 24 hours to confirm the properties of the bacterium. Subsequently, the number of ST viable cells in 1 g of the small intestine content was calculated by multiplying the number of colonies by a dilution factor of the diluted solution.

Moreover, in order to examine the presence or absence of homoserine lactone in the small intestine content, induction of violacein was observed by the same method as that described in the item (8) using CV026 strain. The results are shown in Table 9.

Table 9

|  | Death rate 14 days after infection of ST (%) | Number of viable cells in small intestine content (log CFU/g) (infection index) | Induction of violacein in small intestine content |
|---|---|---|---|
| Example 21 | 12.5 | 1.2 | - |
| Example 22 | 0.0 | 1.1 | - |
| Comparative Example 20 | 50.0 | 5.0 | + |
| Control Example | 50.0 | 5.3 | + |
| +: Induction of violacein was observed. -: Induction of violacein was not observed. | | | |

Both of the death rates of Control Example and the test group of Comparative Example 20 were found to be 50%, and the dead calves exhibited phases such as brownish yellow muddy stool, mucous and bloody stool, dehydration, and astasia at the suckling time. In surviving calves, diarrhea was observed. The death rates of the calves fed with the harmful bacterium control agents of Examples 21 and 22 were found to be as low as 12.5% or less, and in particular, the death rate of the calves fed with the harmful bacterium control agent of Example 22 was found to be 0%. Meanwhile, in the small intestine contents of the calves of Examples 21 and 22, multiplication of ST was inhibited. In addition, induction of bacterial self-induced factors was inhibited. The results reveal that administration of the harmful bacterium control agents containing spores of *Bacillus thuringiensis* of the present invention to calves is effective in prevention/treatment of infection of ST.

(11) Challenge study of *Clostridium* to chick
Feeds obtained by mixing the harmful bacterium control agents of Production Examples 1 to 4 at a concentration of 0.2% by mass with respect to the total mass of a feed for chick (for SD broiler in early stage, manufactured by Nippon Formula Feed Mfg Co., Ltd., a feed supplemented with no antibacterial substance) were used in Examples 23 to 26, respectively. Chicks of the respective 12 groups, each group consisting of 12 chicks hatched from hatching eggs derived from broiler chickens (species: Chunky), were allowed to ingest the feeds of Examples 23 to 26 for 14 days. On the other hand, feeds obtained by mixing the compositions of Comparative Production Examples 1 and 2 at a concentration of 0.2% by mass were used in Comparative Examples 21 and 22 and fed to 6 groups. A feed obtained by mixing lactose instead of a harmful bacterium control agent at a concentration of 0.2% by mass was designated Control Example and fed to 3 groups of the chicks in the same way as above.

When the chicks became 7-day-old, the chicks were orally challenged by *Clostridium perfrigens* (CP) at $1.0 \times 10^6$ CFU/chick, and $1.0 \times 10^7$ CFU/chick, or $1.0 \times 10^8$/chick. And when the chicks became 14-day-old, the cecal content and the stool were collected from each chick. The stool were collected by wiping the cloacae with swabs.

The CP viable cells in the cecal contents were counted by the following method to calculate infection indices and defense indices.

1 g of each cecal content was diluted 10-fold with sterilized phosphate buffered saline, and the solution was mixed well to prepare a sample stock solution- Subsequently, the sample stock solution was serially diluted 10-told with sterilized physiological saline to prepare serially-diluted solutions. The sample stock solution and serially-diluted solutions were separately smeared on a medium for measuring *Clostridia* (manufactured by Nissui Pharmaceutical Co., Ltd.) in an amount of 0.1 ml and cultured anaerobically using Anaeropack Kenki at 35°C for 24 hours, and the number of black colonies multiplied on each plate medium was measured. Moreover, bacterium cells were collected from the colonies and inoculated into CW agar medium supplemented with egg yolk (manufactured by Nissui Pharmaceutical Co., Ltd.), and they were cultured aerobically and anaerobically at 35°C for 24 to 48 hours to confirm their properties.

The number of CP viable cells in 1 g of the cecal content was calculated by multiplying the number of colonies confirmed to be CP by a dilution factor of the diluted solution. Based on the results, infection indices and defense indices were calculated as follows.

Infection index: mean value of logarithms of the numbers of CP viable cells in cecal contents of the respective individuals (mean value of log CFU/g)

Defense index: infection index of control group/infection index of each test group

For each stool collected from the cloacae, qualitative culture was performed by the following method to observe the properties of CP. That is, whether CP colonies were detected or not in the cloacae of each individual was confirmed by: suspending the stool attached to a swab in 10 ml of sterilized phosphate buffered saline to prepare a sample stock solution; smearing the solution in an amount of 0.1 ml on a medium for measuring *Clostridia;* performing anaerobic culture at 35°C for 24 hours using Anaeropack Kenki to evaluate formation of black colonies multiplied on each plate medium; collecting the bacterium from the colonies; inoculating them to CW agar medium supplemented with egg yolk; and performing aerobic culture and anaerobic culture at 354°C for 24 to 48 hours.

In addition, the cell concentrations of the added *Bacillus* bacterium in the stools during the breeding period were measured to calculate mean concentrations for the respective groups.

The results of challenge of CP at $1.0 \times 1.0^6$ CFU are shown in Table 10; the results of challenge of CP at $1.0 \times 10^7$ CFU are shown in Table 11; and the results of challenge of CP at $1.0 \times 10^8$ CFU are shown in Tablet 12.

Table 10

| Challenge of CP at $1.0 \times 10^6$ CFU | | | | |
|---|---|---|---|---|
| | Number of viable cells in cecal content | | Number of individuals whose stools, collected from cloacae, contain CP Number of test chicks (12) | Cell concentration added *Bacillus* bacterium in stool ($\times 10^6$ CFU/g) |
| | Infection index | Defense index | | |
| Example 23 | 3.3 | 2.3 | 6 | 19.5 |
| Example 24 | 3.8 | 2.0 | 4 | 25.3 |
| Example 25 | 3.2 | 2.3 | 6 | 31.4 |
| Example 26 | 3.6 | 2.1 | 5 | 22.1 |
| Comparative Example 21 | 5.3 | 1.4 | 11 | 0.6 |
| Comparative Example 22 | 5.7 | 1.3 | 12 | 0.5 |
| Control Example | 7.5 | - | 12 | 0.0 |

Tabel 11

| Challenge of CP at $1.0 \times 10^7$ CFU | | | | |
|---|---|---|---|---|
| | Number of viable cells in cecal content | | Number of individuals whose stools, collected from cloacae, contain CP Number of test chicks (12) | Cell concentration of added *Bacillus* bacterium in stool ($\times 10^6$ CFU/g) |
| | Infection index | Defense index | | |
| Example 23 | 4.4 | 1.8 | 11 | 15.6 |
| Example 24 | 4.0 | 2.0 | 9 | 29.9 |
| Example 25 | 4.3 | 1.9 | 10 | 27.4 |

(continued)

| Challenge of CP at $1.0 \times 10^7$ CFU | | | | |
|---|---|---|---|---|
| | Number of viable cells in cecal content | | Number of individuals whose stools, collected from cloacae, contain CP Number of test chicks (12) | Cell concentration of added *Bacillus* bacterium in stool ($\times 10^6$ CFU/g) |
| | Infection index | Defense index | | |
| Example 26 | 4.1 | 2.0 | 10 | 24.5 |
| Comparative Example 21 | 6.2 | 1.3 | 12 | 0.4 |
| Comparative Example 22 | 6.6 | 1.2 | 12 | 0.5 |
| Control Example | 8.1 | - | 12 | 0.0 |

Table 12

| Challenge of CP at $1.0 \times 10^8$ CFU | | | | |
|---|---|---|---|---|
| | Number of viable cells in cecal content | | Number of individuals whose stools, collected from cloacae, contain CP Number of test chicks (12) | Cell concentration of added *Bacillus* bacterium in stool ($\times 10^6$ CFU/g) |
| | Infection index | Defense index | | |
| Example 23 | 6.3 | 1.2 | 12 | 22.3 |
| Example 24 | 6.0 | 1.3 | 12 | 18.7 |
| Example 25 | 5.9 | 1.3 | 11 | 30.3 |
| Example 26 | 6.1 | 1.3 | 11 | 17.3 |
| Comparative Examples 21 | 7.7 | 1.1 | 12 | 0.5 |
| Comparative Example 22 | 7.9 | 1.0 | 12 | 0.6 |
| Control Example | 7.8 | - | 12 | 0.0 |

In the stools of the chicks fed with the feeds containing *Bacillus thuringiensis* of Examples 23 to 26 of the present invention, the added *Bacillus* bacterium was detected at concentrations of $1.5 \times 10^7$ CFU/g (gram of raw chick stool) or more, which were significantly larger than those in the stools of the chicks fed with the feeds of Comparative Examples. In the case of challenge of CP at $1.0 \times 10^6$ CFU, the CP infection indices were extremely low, resulting in high defense indices, while in the case of challenge of CP at $1.0 \times 10^7$ CFU, effect of inhibiting multiplication of CP was lowered. In addition, in the case of challenge of CP at $1.0 \times 10^8$ CFU, effect of inhibiting multiplication of CP was not enough. In the case where the chicks fed with the feeds of Comparative Examples 21 and 22 were challenged by a small amount of CP, multiplication of CP was slightly inhibited compared with Control Example.
The results reveal that, when the feeds containing the harmful bacterium control agents of the present invention is given, the *Bacillus thuringiensis* is not killed in the stomach and forms colonies in the intestine. The results further reveal that, in the case where the number of CP cells administered is smaller than the number of viable cells of *Bacillus thuringiensis* in 1 g of the cecal content, the effect of inhibiting CP multiplication is particularly high. Therefore, *Bacillus thuringiensis* is considered to form colonies in the intestine and to competitively exclude CP.
(12) Challenge study of *Clostridiun* bacterium to calf
One-week-old male calves (Holstein) of the respective groups, each group consisting of 8 calves, were bred. Feeds obtained by mixing the harmful bacterium control agents of Production Examples 2 and 3 at a concentration of 2.0% by mass with a mixed feed for calf (Miracle Mate, manufactured by Scientific Feed Laboratory Co., Ltd.) were used in Examples 27 and 28, respectively, while a feed obtained by mixing the composition of Comparative Production Example 1 at a concentration of 2.0% was designated Comparative Example 23. In addition, a feed obtained by adding lactose instead of a harmful bacterium control agent in an equal amount was designated Control Example. These mixed feeds for calf were given to the calves until the calves became 4-week-old. Wen the calves became 2-week-old, the calves were orally challenged by *Clostridium perfingens* (CP) at $3.1 \times 10^7$ CFU/calf. The calves were bred until the calves became 4-week-old, and the death rates of the calves of the respective groups were calculated.

In addition, the small intestine contents were collected, and the numbers of CP viable cells were measured by the same method as above to calculate infection indices and defense indices.

The results are shown in Table 13.

Table 13

| | Death rate 14 days after infection of CP (%) | Number of CP viable cells in small intestine content | |
|---|---|---|---|
| | | Infection index | Defense index |
| Example 27 | 0 | 4.2 | 2.1 |
| Example 28 | 0 | 3.5 | 2.5 |
| Comparative Example 23 | 25 | 6.9 | 1.3 |
| Control Example | 38 | 8.7 | - |

Although the death rate of Control Example and the death rate of the test group of Comparative Example 23 were 38% and 25%, respectively, the death rate of the test groups fed with the feed of Example 27 and 28 were 0%.

In the calves fed with the feeds of Examples 27 and 28, the CP infection indices were very low, and the defense indices were high. The results reveal that administration of a feed containing a harmful bacterium control agent of the present invention is effective in prevention/treatment of CP infectious disease of calves.

(13) Challenge study of *Clostridium* to baby pig

Three-week-old male baby pigs (Large Yorkshire) of the respective groups, each group consisting of 25 pigs, were bred. Feeds obtained by mixing the harmful bacterium control agents of Production Examples 2 and 3 in an amount of 0.5% by mass with respect to the total mass of a feed for baby pig (for SD baby pig synthetic milk in early stage, manufactured by Nippon Formula Feed Mfg Co., Ltd., a feed supplemented with no antibacterial substance) were used in Examples 29 and 30, respectively, and they were given to the pigs until the pigs became 6-week-old. On the other hand, a feed obtained by mixing the composition of Comparative Production Example 1 was designated Comparative Examples 24, and a feed obtained by mixing lactose instead of a harmful bacterium control agent was designated Control Example. The feeds were given to the baby pigs in the same way as above.

When the pigs became 4-week-old, the pigs were orally challenged by *Clostridium perfringens* (CP) at $1.8 \times 10^7$ CFU/pig. The pigs were bred until they became 6-week-old, and the death rates of pigs of the respective groups were calculated. Meanwhile, the small intestine contents were collected, and the numbers of viable cells in the small intestine contents were measured in the same way as above to calculate infection indices and defense indices.

The results are shown in Table 14.

Table 14

| | Death rate 14 days after infection of CP (%) | Number of CP viable cell in small intestine content | |
|---|---|---|---|
| | | Infection index | Defense index |
| Example 29 | 12 | 3.9 | 2.3 |
| Example 30 | 8 | 4.1 | 2.2 |
| Comparative Example 24 | 48 | 7.7 | 1.2 |
| Control Example | 60 | 8.9 | - |

Although the death rate of Control Example and the death rate of the test group fed with the feed of Comparative Example 24 were 60% and 48%, respectively, the death rate of the test group fed with the feed of Example 29 and the death rate of the test group fed with the feed of Example 30 were 12% and 8%, respectively.

In the cases of the test groups fed with the feeds of Examples 29 and 30, the CP infection indices were very low, and the defense indices were high. The results reveal that administration of a feed containing a harmful bacterium control agent of the present invention is effective in prevention/treatment of CP. infectious disease of pigs.

(14) Production of harmful bacterium control agent

The spore formation medium used in the item (3) was used to culture BGSC 4A3, BGSC 4D1, BGSC 4J4, and BGSC 4Q7 at 37°C for 72 hours in a liquid. The resultant culture solutions were centrifuged to collect the bacterial cells. Sterilized water was added thereto so that the density of spores reached $1 \times 10^9$ CFU/ml to produce harmful bacterium control agents, which were used as Examples 31 to 34, respectively. The spore density was measured by: diluting a produced harmful bacterium control agent to an appropriate concentration using sterilized water; heating the agent at 70°C for 30 minutes to kill only vegetative cells; inoculating the cells to a normal agar medium; and counting the number of colonies formed.

On the other hand, compositions produced in the same way as above using *Bacillus coagulans* NBRC12583 and *Bacillus subtilis* NBRC3009 were used in Comparative Examples 25 and 26, respectively.

(15) Rat breeding test

Preliminary feeding of SD rats (Oriental Yeast Co., Ltd., male, initial body weight 120 g) was performed by allowing them to freely ingest water and a feed for experimental animals, MF (manufactured by Oriental Yeast Co., Ltd.) at a breeding temperature of 23°C for one week to confirm the absence of abnormal appearances and behaviors. Subsequently, the spore suspension of Example 31 was diluted with sterilized water to prepare harmful bacterium control agents with spore concentrations of $1 \times 10^6$ CFU/ml, $1 \times 10^7$ CFU/ml, and $1 \times 10^8$ CFU/ml. The harmful bacterium control agent of Example 31 were orally administered to rats of the respective groups, each group consisting of 10 rats, once a day in an amount of 1 ml per rat using a stomach tube. In the same way as above, tests were performed for harmful bacterium control agents containing the spore suspensions of Examples 32 to 34 and for the compositions containing the spore suspensions of Comparative Examples 25 and 26. In addition, a composition containing sterilized water instead of a spore suspension was designated Control Example, which was used in a test in the same way as above.

3 weeks later, the body weights of the rats of the respective groups were measured, and the mean values of weight gains of the groups were calculated. Moreover, one-day's stools were collected, and the concentrations of cells of the added bacterium were measured to calculate the mean values of the respective groups.

The results are shown in Table 15 and 16.

Table 15

| | Average increase in body weight of rats after 3 weeks (g) | | |
|---|---|---|---|
| | Dose $1 \times 10^6$ CFU/rat/day | Dose $1 \times 10^7$ CFU/rat/day | Dose $1 \times 10^8$ CFU/rat/day |
| Example 31 | 156 | 166 | 172 |
| Example 32 | 155 | 169 | 171 |
| Example 33 | 155 | 167 | 169 |
| Example 34 | 157 | 168 | 170 |
| Comparative Example 25 | 146 | 143 | 141 |
| Comparative Example 26 | 142 | 144 | 138 |
| Control Example | 130 | | |

Table 16

| | Bacterial cell concentration of added bacterium in stool (CFU/rat/day) | | |
|---|---|---|---|
| | Dose $1 \times 10^6$ CFU/rat/day | Dose $1 \times 10^7$ CFU/rat/day | Dose $1 \times 10^8$ CFU/rat/day |
| Example 31 | $6.5 \times 10^7$ | $8.2 \times 10^7$ | $9.7 \times 10^7$ |
| Example 32 | $8.6 \times 10^7$ | $8.9 \times 10^7$ | $11.5 \times 10^7$ |
| Example 33 | $8.4 \times 10^7$ | $9.2 \times 10^7$ | $12.1 \times 10^7$ |

(continued)

| | Bacterial cell concentration of added bacterium in stool (CFU/rat/day) | | |
| --- | --- | --- | --- |
| | Dose $1 \times 10^6$ CFU/rat/day | Dose $1 \times 10^7$ CFU/rat/day | Dose $1 \times 10^8$ CFU/rat/day |
| Example 34 | $7.9 \times 10^7$ | $8.3 \times 10^7$ | $10.8 \times 10^7$ |
| Comparative Example 25 cm | $0.03 \times 10^7$ | $0.5 \times 10^7$ | $1.9 \times 10^7$ |
| Comparative Example 26 | $0.06 \times 10^7$ | $0.3 \times 10^7$ | $2.2 \times 10^7$ |
| Control Example | 0 | | |

During the test period, abnormal changes such as death, diseases, and diarrhea were not observed in all the groups. The body weights gain of the rats fed with the harmful bacterium control agents containing spores of *Bacillus thuringiensis* of Examples 31 to 34 was larger than those of the rats fed with the compositions of Comparative Examples 25 and 26. In the stools of the rats fed with the harmful bacterium control agents of Examples 31 to 34, the added bacterium was detected at a concentrations of $6.5 \times 10^7$ CFU/rat/day or more, which is significantly larger than those of Comparative Examples. When the bacterium was administered at a high concentration, the weights gain of the rats became larger. The results reveal that spores of *Bacillus thuringiensis* of the present invention can form colonies in the intestine because they are not killed in the digestive tracts such as stomach and intestines of a rat and can promote the weight gain of the rat.

J (16) Defense test to infection of *Salmonella* bacterium

Preliminary feeding of 7-week-old SPF mice (BAhB/c, Oriental Yeast Co., Ltd., male) was performed by allowing them to freely ingest water and a feed for experimental animals, MF (manufactured by Oriental Yeast Co., Ltd.) at a breeding temperature of 23°C for one week to confirm the absence of abnormal appearances and behaviors. The harmful bacterium control agent containing the spore suspension of Example 31 was orally administered to mice of the respective groups, each group consisting of 10 mice, once a day in an amount of 100 μl per mouse using a stomach tube. In the same way as above, tests were performed for the harmful bacterium control agents containing the spore suspensions of Examples 32 to 34 and the compositions containing the spore suspensions of Comparative Examples 25 and 26. In addition, a composition containing sterilized water instead of a spore suspension was designated Control Example, which was tested in the same way as above.

7 days after the beginning of administration of the spore suspensions, the mice were orally challenged by *Salmonella Typhimurium* (ST) at $1.4 \times 10^4$/mouse using a stomach tube. On day 14 of oral challenge of ST, the cecal contents were collected. The ST viable cells were measured by the following method. 1 g of each cecal content was diluted 10-fold with sterilized phosphate buffered saline, and the solution was mixed well to prepare a sample stock solution. Subsequently, the sample stock solution was serially diluted 10-fold with sterilized physiological saline to prepare serially-diluted solutions. The sample stock solution and serially-diluted solutions were separately smeared on SS agar plate medium "Nissui" (manufactured by Nissui Pharmaceutical Co., Ltd.) and a brilliant green agar plate medium (manufactured by Difco Laboratories) in an amount of 0.1 ml, and culture was performed at 37°C for 24 hours, followed by measurement of the number of typical ST colonies multiplied on each plate medium. Moreover, bacterium cells were collected from the colonies and inoculated into SIM agar medium "Nissui" (manufactured by Nissui Pharmaceutical Co., Ltd.) and TSI agar medium for lysine decarboxylation test "Nissui" (manufactured by Nissui Pharmaceutical Co., Ltd.), and they were cultured at 37°C for 24 hours to confirm their properties. The number of ST viable cells in 1 g of a stool was calculated by multiplying the number of colonies confirmed to be ST by a dilution factor of the diluted solution.

Moreover, the presence or absence of homoserine lactone in the cecal content was examined. In the same way as the method in the item (8) above, an extraction of a cecal content was added dropwise to the surface of the LB medium layered with CV026, and culture was performed, followed by observation of induction of violacein around the regions where the extract was added dropwise.

The results are shown in Table 17.

Table 17

| | Death rate 14 days after infection of ST (%) | Number of ST viable cells in cecal content (log CFU/g) | Induction of violacein |
| --- | --- | --- | --- |
| Example 31 | 0 | 1.9 | - |
| Example 32 | 0 | 1.5 | - |

(continued)

| | Death rate 14 days after infection of ST (%) | Number of ST viable cells in cecal content (log CFU/g) | Induction of violacein |
|---|---|---|---|
| Example 33 | 0 | 1.3 | - |
| Example 34 | 0 | 2.0 | - |
| Comparative Example 25 | 0 | 6.7 | + |
| Comparative Example 26 | 10 | 7.1 | + |
| Control Example | 30 | 8.5 | + |
| +: Induction of violacein was observed. -: Induction of violacein was not observed. | | | |

In the cases of the mice fed with the harmful bacterium control agent containing spores of *Bacillus thuringiensis* of Examples 31 to 34 of the present invention, the numbers of ST viable cells in the cecal contents were very lower than those of the mice fed with the compositions of Comparative Examples 25 and 26, and formation of microgranulomas in the spleen and mesenteric lymph nodes and inflammatory changes in the cecum were not observed at all. In the cases of the mice fed with the compositions of Comparative Examples, multiplication of ST was only slightly inhibited compared with Control Example, and formation of microgranulomas in the spleen and mesenteric lymph nodes and inflammatory changes in the cecum were observed. Meanwhile, with the Control Examples, formation of microgranulomas in the spleen and mesenteric lymph nodes and inflammatory changes in the cecum were observed. The results reveal that administration of spores of *Bacillus thuringiensis* can inhibit multiplication of ST and inhibit expression of bacterial self-induced factors. That is, administration of spores of *Bacillus thuringiensis* is effective in prevention/treatment of intestinal infectious diseases caused by ST.

(17) Defense test to infection of Vibrio bacterium

Preliminary feeding of 7-week-old SPF mice (ICR, Oriental Yeast Co., Ltd., male) was performed in the same way as the item (16) above to confirm the absence of abnormal appearances and behaviors. A harmful bacterium control agent containing the spore suspension of Example 31 was orally administered to mice of the respective groups, each group consisting of 10 mice, once a day in an amount of 100 $\mu$l per mice using a stomach tube. In the same way as above, tests were performed for harmful bacterium control agents containing the spore suspensions of Examples 32 to 34 and the compositions containing the spore suspensions of Comparative Examples 25 and 26. In addition, a composition containing sterilized water instead of a spore suspension was designated Control Example, which was tested in the same way as above.

7 days after the beginning of administration of the spore suspensions, the mice were orally challenged by *Vibrio parahaemolyticus* (VP) at $2.1 \times 10^7$/ mouse using a stomach tube. On day 14 of oral challenge of VP, the small intestine contents were collected. The VP viable cells were measured by the following method. 1 g of each small intestine content was diluted 10-fold with Salt Polymyxin Broth "Nissui" (manufactured by Nissui Pharmaceutical Co., Ltd.), and the solution was mixed well to prepare a sample stock solution. Subsequently, the sample stock solution was serially diluted 10-fold with Salt Polymyxin Broth to prepare serially-diluted solutions. The sample stock solution and serially-diluted solutions were separately smeared on an TCBS agar medium "Nissui" (manufactured by Nissui Pharmaceutical Co., Ltd.) in an amount of 0.1 ml, and culture was performed at 37°C for 18 hours, followed by measurement of the number of typical VP colonies multiplied on each plate medium. The number of VP viable cells in 1 g of the small intestine content was calculated by multiplying the number of colonies by a dilution factor of the diluted solution.

Moreover, the presence or absence of homoserine lactone in the small intestine content was examined. In the same way as the method in the item (8) above, an extraction of a small intestine content was added dropwise to the surface of the LB medium layered with CV026, and culture was performed, followed by observation of induction of violacein around the regions where the extract was added dropwise.

The results are shown in Table 18.

[Table 18]

Table 18

| | Death rate 14 days after infection of VP (%) | Number of VP viable cells in small intestine content (log CFU/g) | Induction of violacein |
|---|---|---|---|
| Example 31 | 10 | 4.7 | - |

## EP 1 967 196 A1

(continued)

|  | Death rate 14 days after infection of VP (%) | Number of VP viable cells in small intestine content (log CFU/g) | Induction of violacein |
|---|---|---|---|
| Example 32 | 0 | 3.9 | - |
| Example 33 | 10 | 4.2 | - |
| Example 34 | 0 | 4.8 | - |
| Comparative Example 25 | 20 | 6.6 | + |
| Comparative Example 26 | 40 | 6.4 | + |
| Control Example | 40 | 7.2 | + |

+: Induction of violacein was observed.
-: Induction of violacein was not observed.

In the cases of the mice fed with the harmful bacterium control agents containing spores of *Bacillus thuringiensis* of Examples 31 to 34 of the present invention, the numbers of VP viable cells in the small intestine contents were lower, and swelling of the small intestine and bowel-content fullness were scarcely observed. On the other hand, in the cases of the mice fed with the bacteria of Comparative Examples 25 and 26, multiplication of VP was only slightly inhibited compared with Control Example, and redly swelling small intestine and bowel-content fullness were observed. The results reveal that administration of the spores of *Bacillus thuringiensis* can inhibit multiplication of VP and inhibit expression of bacterial self-induced factors. That is, administration of spores of *Bacillus thuringiensis* is effective in prevention/treatment of intestinal infectious diseases caused by VP.

(18) Defense test to infection of *Campylobacter* bacterium
Preliminary feeding of 7-week-old SPF mice (BALB/c, Oriental Yeast Co., Ltd., male) was performed in the same way as the item (16) above to confirm the absence of abnormal appearances and behaviors. A harmful bacterium control agent containing the spore suspension of Example 31 was orally administered to mice of the respective groups, each group consisting of 10 mice, once a day in an amount of 100 $\mu$l per mouse using a stomach tube. In the same way as above, tests were performed for harmful bacterium control agents containing the spore suspensions of Examples 32 to 34 and the compositions containing the spore suspensions of Comparative Examples 25 and 26. In addition, a composition containing sterilized water instead of a spore suspension was designated Control Example, which was tested in the same way as above.

7 days after the beginning of administration of the spore suspensions, the mice were orally challenged by *Campylobacter jejuni* (CJ) at $1.7 \times 10^7$/mouse using a stomach tube. On day 14 of oral challenge of CJ, the small intestine contents were collected. The CJ viable cells were measured by the following method. 1 g of each small intestine content was diluted 10-fold with sterilized phosphate buffered saline, and the solution was mixed well to prepare a sample stock solution. Subsequently, the sample stock solution was serially diluted 10-fold with sterilized phosphate buffered saline to prepare serially-diluted solutions. The sample stock solution and serially-diluted solutions were separately smeared on a modified Skirrow medium (manufactured by Nissui Pharmaceutical Co., Ltd.) in an amount of 0.1 ml, and culture was performed at 42°C for 2 or 3 days under microaerobic conditions using Anaeropack Campylo (manufactured by Mitsubishi Gas. Chemical Co., Ltd.) followed by measurement of the number of typical CJ colonies multiplied on each plate medium. The number of CJ cells of in 1 g of the small intestine content was calculated by multiplying the number of colonies confirmed to be CJ by a dilution factor of the diluted solution.

Moreover, the presence or absence of *homoserine* lactone in the small intestine content was examined. In the same way as the method in the item (8) above, an extraction of a small intestine content was added dropwise to the surface of the LB medium layered with CV026, and culture was performed, followed by observation of induction of violacein around the regions where the extract was added dropwise.

The results are shown in Table 19.

[Table 19]

Table 19

| | Death rate 14 days after infection of CJ (%) | Number of CJ viable cells in small intestine content (log CFU/g) | Induction of violacein |
|---|---|---|---|
| Example 31 | 0 | 2.2 | - |
| Example 32 | 0 | 1.4 | - |
| Example 33 | 0 | 1.8 | - |
| Example 34 | 0 | 2.0 | - |
| Comparative Example 25 | 0 | 2.8 | + |
| Comparative Example 26 | 0 | 2.7 | + |
| Control Example | 0 | 3.1 | + |
| +: Induction of violacein was observed.<br>-: Induction of violacein was not observed. | | | |

In the cases of the mice fed with the harmful bacterium control agent containing spores of *Bacillus thuringiensis* of Examples 31 to 34 of the present invention, the concentrations of CJ cells in the small intestine contents were low, and edema and bleeding lesions in the intestine mucosa of the small intestine were not observed. On the other hand, in the cases of the mice fed with the bacteria of Comparative Examples 25 and 26, multiplication of CJ was only slightly inhibited compared with Control Example, and edema and bleeding lesions in the intestine mucosa of the small intestine were observed. Meanwhile, with the Control Example, edema and bleeding lesions in the intestine mucosa of the small intestine were observed. The results reveal that administration of the spores of *Bacillus thuringiensis* can inhibit multiplication of CJ and can inhibit expression of bacterial self-induced factors. That is, administration of the spores of *Bacillus thuringiensis* is effective in prevention/treatment of intestinal infectious diseases caused by CJ.

(19) Challenge study of *Bacillus* to mouse

Preliminary feeding of 7-week-old SPF mice (ICR, Oriental Yeast Co., Ltd., male) was performed by allowing them to freely ingest water and a feed for experimental animals, MF (manufactured by Oriental Yeast Co., Ltd.) at a breeding temperature of 23°C for one week to confirm the absence of abnormal appearances and behaviors. The harmful bacterium control agent of Example 31 was orally administered to mice of the respective groups, each group consisting of 10 mice, once a day in an amount of 100 $\mu$l per mouse for 21 days using a stomach tube. In the same way as above, tests were *performed* for the harmful bacterium control agents of Examples 32 to 34 and the compositions of Comparative Examples 25 and 26. In addition, a composition containing sterilized water instead of a spore suspension was designated Control Example, which was tested in the same way as above.

7 days after the beginning of administration of the spore suspensions, the mice were orally challenged by a kanamycin-resistant mutant of *Bacillus cereus* (BC) (BC Km strain) at $1.0 \times 10^8$ CFU/mouse by orally administrating in an amount of 100$\mu$l of composition using a stomach tube.

The BC Km strain was prepared according to the following procedure. That is, BC was cultured in a broth medium (manufactured by Nissui Pharmaceutical Co., Ltd.) at 37°C for 16 hours. The cells were collected by centrifugation at 4°C, and they were washed with sterilized water once and suspended in sterilized water again. The cells were irradiated with ultraviolet rays at a distance of 20 cm from a UV light and suspended in the broth medium, and culture was performed at 37°C for 4 hours. The culture solution (0.1 $\mu$l) was smeared on a medium (NAKm) obtained by adding kanamycin sulfate (manufactured by Wako Pure Chemical Industries, Ltd.) to a normal agar medium (manufactured by Nissui Pharmaceutical Co., Ltd.) at a concentration of 200 ppm, and culture was performed at 37°C. After culture, colonies formed on NAKm were isolated and used as a BC Km strain.

On day 14 of oral challenge of the BC Km strain (hereinafter, referred to as BC), the death rates of the respective groups were calculated. Meanwhile, the large intestine content was collected, and the number of BC viable cells in the large intestine content was measured by the following method. 1 g of the large intestine content was diluted 10-fold with sterilized phosphate buffered saline, and the whole was mixed well to prepare a sample stock solution. Subsequently, the sample stock solution was serially diluted 10-fold with sterilized physiological saline to prepare serially-diluted solutions. The sample stock solution and serially-diluted solutions were separately applied in an amount of 0.1 ml on a medium obtained by adding kanamycin sulfate (manufactured by Wako Pure Chemical Industries, Ltd.) at a concentration of 200 ppm to NGKG agar medium supplemented with egg yolk (manufactured by Nissui Pharmaceutical Co., Ltd.), and culture was performed at 30°C for 18 hours, followed by measurement of the number of typical BC colonies multiplied on each plate medium. The number of BC viable cells in 1 g of the large intestine content was calculated by multiplying the number of colonies confirmed to be BC by a dilution factor of the diluted solution. Based on the results, the infection indices and defense indices were calculated as follows.

Infection index: Mean value of logarithms of the numbers of BC viable cells in the large intestines of the respective individuals (mean value of log CFU/g)

Defense index: Infection index of control group/Infection Index of each test group

The results are shown in Table 20.

| | Death rate 14 days after infection of BC (%) | Number of BC viable cells in large intestine content | |
|---|---|---|---|
| | | Infection index | Defense index |
| Example 31 | 0 | 4.3 | 1.6 |
| Example 32 | 0 | 4.4 | 1.5 |
| Example 33 | 0 | 4.5 | 1.5 |
| Example 34 | 0 | 4.2 | 1.6 |
| Comparative Example 25 | 0 | 6.5 | 1.0 |
| Comparative Example 26 | 0 | 6.7 | 1.0 |
| Control Example | 0 | 6.8 | - |

In the cases of all the test groups of Examples, Comparative Examples, and Control Example, the death rates were found to be 0%. The test groups fed with the harmful bacterium control agents of Examples 31 to 34 exhibited lower BC infection indices and higher defense indices than those of the test groups fed with the compositions of Comparative Examples. The results reveal that the harmful bacterium control agents of the present invention are effective in prevention/treatment of intestinal infectious diseases caused by BC.

(20) Challenge study of *Staphylococcus* to mouse Preliminary feeding of 7-week-old SPF mice (ICR, Oriental Yeast Co., Ltd., male) was performed in the same way as the item (19) above to confirm the absence of abnormal appearances and behaviors. *Staphylococcus aureus* (SA) was colonized in the intestinal trace of each mouse by the following method of Ida et al. (Takashi Ida, Atsushi Tamura, Katsumi Kawarajo, Jingoro Shimada, Chemotherapy, 42, pp. 923-930 (1994)). First, an aqueous solution of 10 mg/ml ampicillin sodium (manufactured by Wako Pure Chemical Industries, Ltd.) was preliminarily administered to all the mice in an amount of 100 $\mu$l per mouse using a stomach tube once a day for 3 days. On the next day of the completion of administration of ampicillin sodium, a composition obtained by adding SA to the harmful bacterium control agent of Example 31 at $5.0 \times 10^7$ CFU was orally administered to mice of the respective groups, each group consisting of 10 mice, once a day in an amount of 100 $\mu$l per mouse using a stomach tube. From 2 days after the completion of administration of ampicillin sodium, the harmful bacterium control agent of Example 31 was orally administered once a day in an amount of 100 $\mu$l over 13 days. In addition, the harmful bacterium control agents of Examples 32 to 34, the compositions of Comparative Examples 25 and 26, and a composition of Control Example, which was produced by using sterilized water instead of a spore suspension, were orally administered in the same way as above and tested.

On day 14 of the oral challenge of SA, the death rates of the respective groups were calculated. Meanwhile, the large intestine content was collected, and the number of SA viable cells in the large intestine content was measured by the following method. 1 g of the large intestines content was diluted 10-fold with sterilized phosphate buffered saline, and the whole was mixed well to prepare a sample stock solution. Subsequently, the sample stock solution was serially diluted 10-food with sterilized physiological saline to prepare serially-diluted solutions. The sample stock solution and serially-diluted solutions were separately smeared on a salt egg agar medium "Nissui" (manufactured by Nissui Pharmaceutical Co., Ltd.) in an amount of 0.1 ml, and culture was performed at 37°C for 48 hours, followed by measurement of the number of typical SA colonies multiplied on each plate medium. The number of SA viable cells in 1 g of the large intestine content was calculated by multiplying the number of colonies confirmed to be SA by a dilution factor of the diluted solution. Based on the results, the infection indices and defense indices were calculated as follows.

Infection index: Mean value of logarithms of the numbers of SA viable cells in the large intestines of the respective individuals (mean value of log CFU/g)

Defense index: infection index of control group/infection index of each test group

The results are shown in Table 21.

[Table 21]

Table 21

| | Death rate 14 days after infection of SA (%) | Number of SA viable cells in large intestine content | |
| --- | --- | --- | --- |
| | | Infection index | Defense index |
| Example 31 | 0 | 3.2 | 1.6 |
| Example 32 | 0 | 3.0 | 1.7 |
| Example 33 | 0 | 3.1 | 1.6 |
| Example 34 | 0 | 3.0 | 1.7 |
| Comparative Example 25 | 0 | 4.6 | 1.1 |
| Comparative Example 26 | 0 | 4.8 | 1.0 |
| Control Example | 0 | 5.0 | - |

In the cases of all the test groups of Examples, Comparative Examples, and Control Example, the death rates were found to be 0%. The test groups fed with the harmful bacterium control agents of Examples 31 to 34 exhibited lower SA infection indices and higher defense indices than those of the test groups fed with the compositions of Comparative Examples. The results reveal that the harmful bacterium control agents of the present invention are effective in prevention/treatment of intestinal infectious diseases caused by SA.

(21) Challenge study of *Streptococcus* to mouse

Preliminary feeding of 7-week-old SPF mice (ICR, Oriental Yeast Co., Ltd., male) was performed in the same way as the item (19) above to confirm the absence of abnormal appearances and behaviors. The harmful bacterium control agent of Example 31 was filled in a water feeder, and mice of the respective groups, each group consisting of 10 mice, were allowed to freely ingest water for 21 days. The water feeder was exchanged every day to exchange the agent for a fresh harmful bacterium control agent (spore suspension)- In the same as above, the harmful bacterium control agents of Examples 32 to 34 and the compositions of Comparative Examples 25 and 26 were administered. In addition, a composition containing sterilized water instead of a spore suspension was designated Control Example and administered in the same way as above.

7 days after the beginning of administration of the spore suspensions, $1.1 \times 10^8$ CFU of *Streptococcus suis* (SS) was instilled into a pre-sterilized swab in an amount of 100 $\mu$l of composition, and the mice were orally challenged by the SS by slowly rotating the swab in the oral cavity of each mouse for 30 seconds. After challenge of SS, the mice were observed every day to calculate the ratios of individuals that developed neurological symptoms such as torticollis and turning behavior and dead individuals. For a dead individual, the blood was aseptically collected from the heart while the lung, liver, spleen, kidney, and brain-spinal fluid were aseptically collected. Moreover, a pre-sterilized swab was used to collect throat tissues. On day 14 of administration of SS, the respective tissues were collected from survival mice in the same way as above. Immediately after collection, the tissues were cultured at 37°C for 24 hours in Todd Hewitt broth (manufactured by Oxoid). A medium where multiplication was observed was further inoculated into a heart infusion agar medium (manufactured by manufactured by Nissui Pharmaceutical Co., Ltd.) supplemented witch 5% by volume of sheep blood, and culture was performed at 37°C for 24 to 48 hours to confirm the properties. A tissue that was confirmed to contain SS was judged as positive, and the ratio of individuals judged as positive for each tissue was calculated and defined as an SS positive ratio.

Meanwhile, the brain was fixed with a 10% buffered formalin solution, and paraffin sections were prepared according to a conventional method and subjected to hematoxylin-eosin staining, and histological findings were examined to calculate the incidence rate of meningitis.

The results are shown in Table 22.

Table 22

| | Necrological symptom incidence/ death rate (%) | SS-positive ratio (%) | | | | | | | Incidence rate of meningitis (%) |
| --- | --- | --- | --- | --- | --- | --- | --- | --- | --- |
| | | Heart blood | Lung | Liver | Spleen | Kidney | Brain-spinal fluid | Throat | |
| Example 31 | 20 | 20 | 20 | 20 | 20 | 20 | 20 | 20 | 20 |

(continued)

| | Necrological symptom incidence/ death rate (%) | SS-positive ratio (%) | | | | | | | Incidence rate of meningitis (%) |
|---|---|---|---|---|---|---|---|---|---|
| | | Heart blood | Lung | Liver | Spleen | Kidney | Brain-spinal fluid | Throat | |
| Example 32 | 30 | 30 | 30 | 20 | 20 | 30 | 30 | 30 | 30 |
| Example 33 | 20 | 20 | 20 | 20 | 20 | 20 | 20 | 20 | 20 |
| Example 34 | 30 | 30 | 30 | 30 | 30 | 30 | 30 | 30 | 30 |
| Comparative Example 25 | 50 | 50 | 40 | 40 | 40 | 40 | 50 | 50 | 50 |
| Comparative Example 26 | 40 | 40 | 40 | 40 | 40 | 40 | 40 | 40 | 40 |
| Control Example | 50 | 50 | 50 | 50 | 50 | 50 | 50 | 50 | 50 |

In the cases of the test groups of Examples 31 and 33, the death rates were as low as 20%. Meanwhile, in the cases of the test groups of Examples 32 and 34, the death rates were 30%. Although the SS-positive ratios of the respective groups were different depending on the type of the tissues, in the cases of the test groups of Examples 31 and 33, the SS-positive ratios were as low as 20% in all the tissues. In addition, in the case of the test group of Example 32, the SS-positive ratios were as low as 20% in the liver and spleen and 30% in the other tissues. In the case of the test group of Example 34, the SS-positive ratios of the respective tissues were relatively high (30%) On the other hand, in the case of the test group of Comparative examples, the SS-positive ratios in the respective tissues were as high as 40 to 50%, and infection of SS was only slightly inhibited compared with Control Example. In addition, in the cases of the test groups of Examples 31 to 34, the incidence rates of meningitis were 20 to 30%, and the values were lower than those of the test groups of Comparative Examples.
The results reveal that administration of the harmful bacterium control agents of the present invention is effective in prevention/treatment of infectious diseases caused by SS and in lowering of incidence rates of nervous disorders and meningitis.

(22) Pharmaceutical containing *Bacillus thuringiensis*

(A) Ointment

*Bacillus thuringiensis* BGSC 4A3, BGSC 4D1, BGSC 4J4, and BGSC 4Q7 were subjected to liquid culture at 37°C for 72 hours using the spore formation medium described in the item (3). The resultant culture solution was centrifuged to collect the bacteria cells. The resultant bacterial cells were freeze-dried and pulverized, and D(+)trehalose dihydrate (special grade, manufactured by Wako Pure Chemical Industries, Ltd.) was added at a spore density of $1 \times 10^9$ CFU/g. 1 g of the resultant trehalose containing spores of a bacterium *Bacillus thuringiensis* and 5 g of a polyethylene resin/liquid paraffin ointment (trade name: Plastibase, manufactured by Taisho Pharmaceutical Co., Ltd.) heated to 60°C were added, and the whole was mixed well. Then, the total weight was adjusted to 100 g by further adding the polyethylene resin/liquid paraffin ointment, and the whole was kneaded uniformly, to thereby prepare ointments of Examples 35 to 38. The spore densities of the ointments of Examples 35 to 38 were found to be $1.1 \times 10^7$ CFU/g, $1.2 \times 10^7$ CFU/g, $1.0 \times 10^7$ CFU/g, and $1.1 \times 10^7$ CFU/g, respectively. The spore density was measured by: diluting the ointment to an appropriate concentration with sterilized water containing 0.1% by mass of Tween 80 (registered trademark); heating the solution at 70°C for 30 minutes to kill only vegetative cells; inoculating the solution into a normal agar medium; and measuring the number of colonies formed. On the other hand, ointments were produced using *Bacillus coagulans* NBRC12583 and *Bacillus subtilis* NBRC3009 in the same way as above and designated Comparative Examples 27 and 28, respectively. The spore densities of the resultant ointments of Comparative Examples 27 and 28 were found to be $1.0 \times 10^7$ CFU/g and $1.1 \times 10^7$ CFU/g, respectively. Meanwhile, an ointment produced in the same way as above using D(+)trehalose dihydrate supplemented with no *Bacillus* bacterium was designated Control Example.

Preliminary feeding of SD rats (Oriental Yeast Co., Ltd., male, initial body weight 120 g) of the respective groups, each group consisting of 10 rats, was performed by allowing them to freely ingest water and a feed for experimental animals, MF (manufactured by Oriental Yeast Co., Ltd.) at a breeding temperature of 23°C for one week to confirm the absence of abnormal appearances and behaviors. Each rat was anaesthetized with pentobarbital, and the right femur was dehaired, fixed on a fixed base, and compressed by the bottom of a disposable syringe (diameter 16 mm) for 6 hours a day over 6 days (pressure: 1.0 kg/cm$^2$). A thin silicon cushion was placed between the bottom of the syringe and the femur of the rat.

After completion of the compression test, the ointments of Examples, Comparative Examples, and Control Example obtained above were administered to the pressure sore sites in an amount of 100 mg per day to evaluate the drug efficacy of each ointment. The drug efficacy was evaluated by visual observation and histopathological observation. Visual observation was performed by measuring the major and minor axes using a caliper to measure changes in areas of the pressure sore sites with respect to the number of days elapsed and the number of days for treatment.

First, a product of the major and minor axes of a pressure sore site on day 2 of development of a pressure sore was defined as 100%, and the ratios (area ratios) of the product of the major and minor axes measured after 3 days of the development of the pressure sore were calculated by the following expression.

Area ratio (%) = (major axis × minor axis of pressure sore site [on the day of observation]) × 100/(major axis × minor axis of pressure sore site [on day 2 of development of pressure sore])

The area ratio (y) was plotted against the number of days elapsed (x) to create a curve (f(x)), and an approximate area of a region represented by

$$0 \leq y \leq f(x)$$

and

$$5 \leq x \leq 20$$

was calculated by the following expression and used as an index of curing process. That is, the larger the number of days required for reducing the size of a pressure sore site, the larger the index of curing process.

$$\text{Index of curing process} = \sum_{n=5}^{19} \frac{\text{Area ratio [On day n] + Area ratio [On day n+1]}}{2}$$

In addition, the number of days required for completing epithelium formation at a pressure sore site to cure the sore is defined as the number of days for curing.

The results are shown in Table 23.

Table 23

|  | Number of days for curing | Index of curing process |
|---|---|---|
| Example 35 | 16.7 | 435 |
| Example 36 | 16.8 | 427 |
| Example 37 | 15.9 | 431 |
| Example 38 | 16.3 | 429 |
| Comparative Example 27 | 18.6 | 514 |
| Comparative Example 28 | 19.5 | 521 |
| Control Example | 21.7 | 576 |

As is clear from Table 23, in the cases of applying the ointments of Examples 35 to 38, the numbers of days for curing were smaller than those in the cases of applying the ointments of Comparative Examples 27 and 28. In addition, the indices of curing process were found to be small, and high curing rates were achieved at an early date.

On days 5 and 10 after the beginning of ointment application, the skin of each pressure sore site was removed and fixed with a 10% neutral buffered formalin solution, and paraffin sections were created in accordance with a conventional method and subjected to hematoxylin-eosin staining, followed by histopathological observation. In the histopathological observation, regeneration of the epithelial tissues of residual hair follicles on day 5 of the beginning of the application was observed, and the peeling of scab from peripheral portions was observed. The granulation tissue formation was clearly observed in the lower layer of the regenerated epithelium, and the tissue repair was clearly observed compared with Comparative Examples 27 and 28. In addition, on day 10 of the beginning of the application, elongation of the epithelium from peripheral portions was clearly observed, and the cell wetting was observed only in the upper layer. Moreover, fiber formation was observed in the regenerated epithelium, and the site was getting better. On the other hand, in the peripheral portions of the pressure sore sites where the ointments of Comparative Examples 27 and 28 were applied, coating with the regenerated epithelium was observed, and defective parts were covered with thick scab, resulting in inhibition of epithelium elongation. The results reveal that the ointments containing *Bacillus thuringiensis* of Examples 35 to 38 were found to have ability to promote curing of damages.

(B) Antiflatulent

*Bacillus thuringiensis* BGSC 4D1 was subjected to liquid culture at 37°C for 72 hours in the above-described spore formation medium. The resultant culture solution was centrifuged to collect cells. The resultant cells were freeze-dried and pulverized, and D(+)trehalose dihydrate (special grade, manufactured by Wako Pure Chemical Industries, Ltd.) was added at a spore density of $2 \times 10^7$ CFU/g. The resultant trehalose containing spores of a bacterium *Bacillus thuringiensis* (50 parts by mass) was mixed with potato starch (50 parts by mass), and a mixture was formed into 350 mg of tablets, to thereby yield an antiflatulent, which was designated Example 39. The spore density of the antiflatulent of Example 39 was found to be $1.1 \times 10^7$ CFU/g. The spore density was measured by: diluting the antiflatulent to an appropriate concentration with sterilized water; heating the solution at 70°C for 30 minutes to kill only vegetative cells; inoculating the solution into a normal agar medium; and measuring the number of colonies formed. On the other hand, an antiflatulent was produced using *Bacillus coagulants* NBRC12583 in the same way as above and designated Comparative Example 29. The spore density of the resultant antiflatulent of Comparative Example 29 was found to be $1.2 \times 10^7$ CFU/g. Meanwhile, a composition produced using D(+)trehalose dihydrate supplemented with no *Bacillus* bacterium in the same way as above was designated Control Example.

(a) Loose stool amelioration test

For adults with loose stool, effects of an antiflatulent containing *Bacillus thuringiensis* on stool properties were examined. 11 men in their forties and 7 men in their fifties were allowed to ingest 3 tablets of the antiflatulent of Example 39 after breakfast, lunch, and dinner for 2 weeks. During the next 2 weeks, they were allowed to ingest the composition of Control Example. During the further next 2 weeks, they were allowed to ingest the antiflatulent of Comparative Example 29. A period of 2 weeks before the beginning of the ingestion period was defined as a non-ingestion period, and a questionnaire was conducted on their stool properties for the non-ingestion period and the ingestion periods. That is, the shape and hardness of each stool were recorded according to the following criteria.

Shape of stool: 1. watery, 2.muddy, 3. half-kneaded-like, 4. banana-like, 5. hard, 6. ball-like

Hardness: 1. very soft, 2. soft, 3. normal, 4. hard, 5. very hard

The results are shown in Table 24.

Table 24

| Stool property | Before ingestion | Example 39 | Control Example | Comparative Example 29 |
|---|---|---|---|---|
| Shape | 2.9 | 3.8 | 3.1 | 3.3 |
| Hardness | 2.2 | 2.7 | 2.4 | 2.5 |

Ingestion of the antiflatulent of Example 39 improved the shape retention for the shape and hardness of stool. On the other hand, in the case of ingestion of the antiflatulent of Comparative Example 29, the shape and hardness of each stool, and the feeling were found to be about the same as those in the case of Control Example.

(b) Constipation amelioration test

For adults with constipation, effects of antiflatulents containing spores of *Bacillus thuringiensis* on stool properties were examined. 17 women in their thirties and 5 women in their forties were allowed to ingest 3 tablets of the antiflatulent of Example 39 after breakfast, lunch, and dinner for 2 weeks. During the next 2 weeks, they were allowed to ingest the composition of Control Example. During the further next 2 weeks, they were allowed to ingest the antiflatulent of Comparative Example 29. A period of 2 weeks before the beginning of the ingestion period was defined as a non-ingestion period, and a questionnaire was conducted on their stool properties for the non-ingestion period and the ingestion periods. The numbers of times of bowel movement, stool shapes, and feelings were recorded according to the following criteria.

The number of times of bowel movement: times/week

Shape of stool: 1. watery, 2. muddy, 3. half-kneaded-like, 4. banana-like, 5. hard, 6. ball-like

Feeling: 1. very bad, 2. bad, 3. normal, 4. good, 5. very good

The results are shown in Table 25.

Table 25

| Stool property | Before ingestion | Example 39 | Control Example | Comparative Example 29 |
|---|---|---|---|---|
| Number of times of bowel movement | 3.0 | 4.1 | 3.3 | 3.5 |
| Shape | 5.0 | 4.1 | 4.3 | 4.3 |
| Feeling | 3.4 | 3.6 | 3.4 | 3.5 |

Ingestion of the antiflatulent of Example 39 improved the number of times of bowel movement, stool shapes, and feelings. On the other hand, in the case of ingestion of the antiflatulent of Comparative Example 29, the number of times of bowel movement, stool shapes, and feelings were found to be about the same as those of Control Example. The results reveal that an antiflatulent containing spores of *Bacillus thuringiensis* can ameliorate bowel movement and stool properties.

(23) Food containing *Bacillus thuringiensis*

*Bacillus thuringiensis* BGSC 4D1 was subjected to liquid culture at 37°C for 24 hours in the above-described spore formation medium. Minimal soybeans were immersed in tap water at 44°C overnight and sterilized in a pressure vessel at 121°C for 30 minutes. Into the resultant sterilized soybeans was inoculated a solution obtained by diluting the above-described *Bacillus* culture solution to 1,000-fold in an amount of 2 ml with respect to 100 g of the sterilized soybeans, and culture was performed at 37°C for 48 hours. Moreover, aging in cold storage was performed at 4°C for 72 hours, and a soybean culture product was designated Example 40. The spore density was found to be $2.9 \times 10^9$ CFU/g. The spore density was measured by: adding 90 ml of sterilized water to the soybean culture product; homogenizing the mixture using a homogenizer (manufactured by NISSEI Corporation) at 10,000 rpm for 2 minutes; diluting the homogenate to an appropriate concentration with sterilized water; heating the solution at 70°C for 30 minutes to kill only vegetative cells; inoculating the solution into a normal agar medium; and measuring the number of colonies formed. A soybean culture product was produced using *Bacillus subtills* NBRC3009 in the same way as above and designated Comparative Example 30. The spore density was found to be $1.0 \times 10^9$ CFU/g. Meanwhile, sterilized soybeans supplemented with no *Bacillus* bacterium was designated Control Example.

(a) Loose stool amelioration test

For adults with loose stool, effects of a food containing *Bacillus thuringiensis* on stool properties were examined. 11 men in their forties and 7 men in their fifties were allowed to eat 100g of the food of Example 40 per day for two weeks. During the next 2 weeks, they were allowed to eat the food of Control Example. During the further next 2 weeks, they were allowed to eat the food of Comparative Example 30. A period of 2 weeks before the eating period was set as non-eating period, and a questionnaire was conducted on their stool properties. That is, the shape and hardness of each stool were recorded according to the following criteria.

Shape of stool: 1. watery, 2.muddy, 3. half-kneaded-like, 4. banana-like, 5. hard, 6. ball-like

Hardness: 1. very soft, 2. soft, 3. normal, 4. hard, 5. very hard

The results are shown in Table 26.

Table 26

| Stool property | Before eating | Example 40 | Control Example | Comparative Example 30 |
|---|---|---|---|---|
| Shape | 3.1 | 3.7 | 3.0 | 3.6 |
| Hardness | 2.5 | 3.0 | 2.2 | 2.4 |

Ingestion of the food of Example 40 improved the shape retention for the shape and hardness of stool. On the other hand, in the case of ingestion of the food of Comparative Example 30, the shape and hardness of each stool, were found to be about the same as those in the case of Control Example.

(b) Constipation amelioration test

For adults with constipation, effects of food containing *Bacillus thuringiensis* on stool properties were examined. 17 women in their thirties and 5 women in their forties were allowed to eat 100 g of the food of Example 40 per day for 2 weeks. During the next 2 weeks, they were allowed to eat the food of Control Example. During the further next 2 weeks, they were allowed to eat the food of Comparative Example 30. A period of 2 weeks before the eating period was set as non-eating period, and a questionnaire was conducted on their stool properties. The numbers of times of bowel movement, stool shapes, and feelings were recorded according to the following criteria.

The number of times of bowel movement: times/week

Shape of stool: 1. watery, 2. muddy, 3. half-kneaded-like, 4. banana-like, 5. hard, 6. ball-like

Feeling: 1. very bad, 2. bad, 3. normal, 4. good, 5. very good

The results are shown in Table 27.

Table 27

| Stool property | Before eating | Example 40 | Control Example | Comparative Example 30 |
|---|---|---|---|---|
| Number of times of bowel movement | 3.5 | 4.7 | 3.2 | 3.5 |
| Shape | 4.3 | 4.1 | 4.5 | 4.5 |
| Feeling | 3.5 | 3.9 | 3.5 | 3.6 |

Eating of the food of Example 40 improved the number of times of bowel movement, stool shapes, and feelings. On the other hand, in the case of eating of the food of Comparative Example 30, the number of times of bowel movement, stool shapes, and feelings were found to be about the same as those of Control Example.

The results reveal that a food containing spores of *Bacillus thuringiensis* can ameliorate bowel movement and stool properties.

Industrial Applicability

If a harmful bacterium control agent of the present invention is administered to an animal, *Bacillus thuringiensis* may multiply in the intestine to inhibit multiplication of a harmful bacterium and to inactivate bacterial self-induced factors, resulting in improvement of the balance of intestinal flora or prevention/treatment of infectious diseases. In particular, *Bacillus thuringiensis* contained in the harmful bacterium control agent of the present invention is considered to have some effects such as antagonistic action, bacterial interference, barrier effect, inhibition of colonization to cells of the intestinal tract, and competitive exclusion.

A harmful bacterium control agent of the present invention can be used in drugs for preventing/treating infectious diseases, foods for improving the balance of intestinal flora, and feeds for promoting growth of livestock animals.

**Claims**

1. A harmful bacterium control agent, comprising *Bacillus thuringiensis.*

2. A harmful bacterium control agent according to claim 1, wherein the *Bacillus thuringiensis* has a bile acid resistance.

3. A harmful bacterium control agent according to claim 1 or 2, wherein the *Bacillus thuringiensis* further has an ability of bacterial self-induced factor inactivation.

4. A harmful bacterium control agent according to any tone of claims 1 to 3, wherein the *Bacillus thuringiensis* is a *Bacillus thuringiensis* subsp. *thuringiensis* BGSC 4A3 strain, a *Bacillus thuringiensis* subsp. *kurstaki* BGSC 4D1 strain, a *Bacillus thuringiensis* subsp. *aizawai* BGSC 4J4 strain, a *Bacillus thuringiensis* subsp. *israelensis* BGSC 4Q7 strain, or mutants thereof.

5. A harmful bacterium control agent according to any one of claims 1 to 4, wherein the harmful bacterium is a pathogen causing an infectious disease.

6. A harmful bacterium control agent according to any one of claims 1 to 5, wherein the pathogen is an infectious disease caused by a pathogen belonging to a gram-negative bacterium.

7. A harmful bacterium control agent according to claim 6, wherein the pathogen belonging to a gram-negative bacterium comprises at least any one of bacteria each belonging to genus *Salmonella, Campylobacter, Vibro, Yersinia*, *Aeromonas, Pseudomonas, Escherichia coli,* and *Shigella.*

8. A harmful bacterium control agent according to any one of claims 1 to 5, wherein the pathogen is an infection disease caused by a pathogen belonging to a gram-positive bacterium.

9. A pathogen control agent according to claim 8, wherein the pathogen belonging to a gram-positive bacterium comprises at least any one of bacteria each belonging to genus *Clostridium, Bacillus, Staphylococcus, Streptococcus,* and *Listeria.*

10. A pharmaceutical, comprising the harmful bacterium control agent according to any one of claims 1 to 9.

11. A pharmaceutical according to claim 10, wherein the pharmaceutical is for preventing or treating an infectious disease caused by a pathogen.

12. A pharmaceutical according to claim 10 or 11, wherein the pharmaceutical is for human.

13. A pharmaceutical according to claim 10 or 11, wherein the pharmaceutical is for a non-human animal.

14. A food, comprising the harmful bacterium control agent according to any one of claims 1 to 9.

15. A feed, comprising the harmful bacterium control agent according to any one of claims 1 to 9.

16. A feed according to claim 15, wherein the feed is for preventing or treating an intestinal infectious disease of an animal.

17. A method of breeding an animal, comprising feeding the feed according to claim 15 or 16 to an animal.

## INTERNATIONAL SEARCH REPORT

| International application No. |
| --- |
| PCT/JP2006/319957 |

A. CLASSIFICATION OF SUBJECT MATTER
*A61K35/74*(2006.01)i, *A01N63/00*(2006.01)i, *A23K1/16*(2006.01)i, *A23L1/30*
(2006.01)i, *A61P1/10*(2006.01)i, *A61P31/04*(2006.01)i, *A61P43/00*(2006.01)i,
*C12N1/20*(2006.01)i, *C12R1/07*(2006.01)n
According to International Patent Classification (IPC) or to both national classification and IPC

B. FIELDS SEARCHED

Minimum documentation searched (classification system followed by classification symbols)
A61K35/74, A01N63/00, A23K1/16, A23L1/30, A61P1/10, A61P31/04, A61P43/00,
C12N1/20, C12R1/07

Documentation searched other than minimum documentation to the extent that such documents are included in the fields searched
Jitsuyo Shinan Koho       1922-1996    Jitsuyo Shinan Toroku Koho    1996-2006
Kokai Jitsuyo Shinan Koho   1971-2006    Toroku Jitsuyo Shinan Koho    1994-2006

Electronic data base consulted during the international search (name of data base and, where practicable, search terms used)
BIOSIS(STN), CAplus(STN), EMBASE(STN), MEDLINE(STN), WPIDS(STN),
JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2000-103740 A  (Kabushiki Kaisha Taki<br>Seibutsu Kagaku Kenkyusho),<br>11 April, 2000 (11.04.00),<br>Full text; Claims; Par. No. [0012]; examples<br>(particularly, example 4, comparison section 1)<br>(Family: none) | 1-17<br>1-17 |
| X<br>Y | JP 2000-143521 A  (Kabushiki Kaisha Taki<br>Seibutsu Kagaku Kenkyusho),<br>23 May, 2000 (23.05.00),<br>Full text; Claims; Par. No. [0012]; examples<br>(Family: none) | 1-17<br>1-17 |

☒ Further documents are listed in the continuation of Box C.  ☐ See patent family annex.

| | |
| --- | --- |
| *     Special categories of cited documents:<br>"A"   document defining the general state of the art which is not considered   to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>     13 November, 2006 (13.11.06) | Date of mailing of the international search report<br>     21 November, 2006 (21.11.06) |
| --- | --- |
| Name and mailing address of the ISA/<br>     Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2006/319957 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | YUDINA, TG ET AL. 'ANTIMICROBIAL ACTIVITY OF CRY- AND CYT-PROTEINS FROM BACILLUS THURINGIENSIS SSP. ISRAELENSIS.' CAN. J. MICROBIOL., (2003) 49(1) P.37-44, full text; ABSTRACT | 1-17 |
| Y | REVINA, LP ET AL. 'NOVEL ANTIBACTERIAL PROTEINS FROM ENTOMOCIDAL CRYSTALS OF BACILLUS THURINGIENSIS SSP. ISRAELENSIS.' CAN. J. MICROBIOL., (2005 FEB.) 51(2) P.141-148 | 1-17 |
| Y | CHERIF, A. ET AL. 'THURICIN 7: A NOVEL BACTERIOCIN PRODUCED BY BACILLUS THURINGIENSIS BMG1.7, A NEW STRAIN ISOLATED FROM SOIL.' LETT. AAPPL. MICROBIOL., (2001) 32(4) P.243-247 Full text; ABSTRACT, INTRODUCTION, Table1, P.246 right column, lines 6 to 19 | 1-17 |
| Y | CHERIF, A. ET AL. 'DETECTION AND CHARACTERIZATION OF THE NOVEL BACTERIOCIN ENTOMOCIN 9, AND SAFETY EVALUATION OF ITS PRODUCER, BACILLUS THURINGIENSIS SSP. ENTOMOCIDUS HD9.' J. APPL. MICROBIOL., (2003) 95(5) P.990-1000, Full text; ABSTRACT, Table 2, page 996, left column, middle part to right column, line 13, page 998, right column, lines 1 to 17 | 1-17 |
| Y | JP 2000-506739 A (The Regents of the University of California), 06 June, 2000 (06.06.00), Full text; Claim 10 & WO 97/39623 A2     & AU 9740590 B & EP 896619 A2 | 1-17 |
| P,X | JP 2005-289878 A (Nakamura Sangyo Kabushiki Kaisha), 20 October, 2005 (20.10.05), Claims; Par. Nos. [0003] to [0004], [0016] to [0032] (Family: none) | 1,5 |
| X | JP 2001-524806 A (Agraquest, Inc.), 04 December, 2001 (04.12.01), Full text; Claims; examples & WO 98/21965 A1     & AU 1998/54498 A1 & US 5919447 A     & EP 938261 A1 & US 6077506 A | 1,5 |
| A | US 5981698 A (CYBERCHEMICS INC), 09 November, 1999 (09.11.99), Full text (Family: none) | 1-17 |

Form PCT/ISA/210 (continuation of second sheet) (April 2005)

**INTERNATIONAL SEARCH REPORT**

International application No.

PCT/JP2006/319957

C (Continuation).    DOCUMENTS CONSIDERED TO BE RELEVANT

| Category* | Citation of document, with indication, where appropriate, of the relevant passages | Relevant to claim No. |
|---|---|---|
| A | Masami MOCHIZUKI, "Kaisetsu Seibutsu Noyaku no Doko", Idemitsu Technical Report, 15 October, 1997 (15.10.97), 40(5) pages 121 to 125, full text | 1-17 |

Form PCT/ISA/210 (continuation of second sheet) (April 2005)

**EP 1 967 196 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005507670 A
- JP 5268944 A
- WO 96024659 A
- JP 2004523241 A
- JP 2003504028 A
- JP 2005508617 A
- JP 11285378 A
- JP 2002034582 A
- US 5468483 A

**Non-patent literature cited in the description**

- Bacillus Spores as Probiotic Products for Human Use, Bacterial spore formers. **SENESI, S.** Horizon Bioscience. 2004, 131-141
- **HYRONIMUS, B. et al.** *International Journal of Food Microbiology,* 2000, vol. 61, 193-197
- **KAPLAN,H. et al.** *Journal of Bacteriology,* 1985, vol. 163, 1210-1214
- **TATEDA,K.** *The Lung Perspectives,* 2005, vol. 13, 261-265
- **YI-HU DONG et al.** *Applied and Environmental Microbiology,* 2004, vol. 70, 954-960
- **BERGEY'S.** Manual of Determinative Bacteriology. 1994
- **MCCLEAN,K. et al.** *Microbiology,* 1997, vol. 143, 3073-3711
- **SCHAEFFER, P. ; J. MILLET ; J.P. AUBERT.** *Proceedings of the National Academy of Science, US,* 1965, vol. 54, 704-711
- **MCCLEAN, K. et al.** *Microbiology,* 1997, vol. 143, 3703-3711
- *Proceedings of the National Academy of Sciences, US,* 1965, vol. 54, 704-711
- **TAKASHI IDA ; ATSUSHI TAMURA ; KATSUMI KAWARAJO ; JINGORO SHIMADA.** *Chemotherapy,* 1994, vol. 42, 923-930